# FASCICULE DE BREVET EUROPEEN

(11) **EP 1 648 494 B1**
(45) Date de publication et mention de la délivrance du brevet: **22.02.2012**
(21) Numéro de dépôt: 04786012.7
(22) Date de dépôt: 30.07.2004
(51) Int. Cl.: A61K 38/18, A61P 35/00, A61P 17/00, A61P 19/02, A61P 27/02, A61P 37/06

(54) **Agent anti-angiogenique et son utilisation dans le cadre du traitement des cancers**
Anti-angiogenetisches Mittel und seine Verwendung bei der Krebsbehandlung
Anti-angiogenetic agent and its use in cancer treatment

(30) Priorité: 01.08.2003 FR 0309506
(43) Date de publication de la demande: 26.04.2006
(73) Titulaire: CENTRE NATIONAL DE LA RECHERCHE SCIENTIFIQUE (CNRS), 75794 Paris Cedex 16 (FR); INSTITUT NATIONAL DE LA SANTE ET DE LA RECHERCHE MEDICALE (INSERM), 75654 Paris Cédex 13 (FR)
(72) Inventeur: PLOUET, Jean, F-75014 Paris (FR); LAURENT-BEUBRY, Maryvonne, F-91300 Massy (FR); MARTINERIE-KRYCEVE, Cécile, F-91120 Palaiseau (FR)
(74) Mandataire: Grosset-Fournier, Chantal Catherine
(86) Numéro de dépôt international: PCT/FR2004/002050
(87) Numéro de publication internationale: WO 2005/011725

(56) Documents cités:
- EP-A- 1 382 347
- PERBAL BERNARD ET AL: "The C-terminal domain of the regulatory protein NOVH is sufficient to promote interaction with fibulin 1C: A clue for a role of NOVH in cell-adhesion signaling" PROCEEDINGS OF THE NATIONAL ACADEMY OF SCIENCES OF THE UNITED STATES OF AMERICA, vol. 96, no. 3, 2 février 1999 (1999-02-02), pages 869-874, XP002322020 ISSN: 0027-8424
- M.L. IRUELA-ARISPE ET AL.: "INHIBITION OF ANGIOGENESIS BY THROMBOSPONDIN-1 IS MEDIATED BY 2 INDEPENDENT REGIONS WITHIN THE TYPE 1 REPEATS" CIRCULATION, AMERICAN HEART ASSOCIATION, DALLAS, TX, US, vol. 100, no. 13, 28 septembre 1999 (1999-09-28), pages 1423-1431, XP000923386 ISSN: 0009-7322
- INOKI ISAO ET AL: "Connective tissue growth factor binds vascular endothelial growth factor (VEGF) and inhibits VEGF-induced angiogenesis" FASEB JOURNAL, [Online] 14 février 2001 (2001-02-14), pages 1-27, XP002321942 Extrait de l'Internet: URL:http://www.fasebj.org/cgi/reprint/01-0 332fjev1?maxtoshow=&HITS=10&hits=10&RESULT FORMAT=&fulltext=inoki&searchid=1111582791 442_665&stored_search=&FIRSTINDEX=0&volume =16&issue=2&journalcode=fasebj> [extrait le 2005-03-21] & INOKI ISAO ET AL: THE FASEB JOURNAL, vol. 16, no. 2, février 2002 (2002-02), pages 219-221, ISSN: 1530-6860 cité dans la demande
- N. GUPTA ET AL.: "INHIBITION OF GLIOMA CELL GROWTH AND TUMORIGENIC POTENTIAL BY CCN3 (NOV)" JOURNAL OF CLINICAL PATHOLOGY: MOLECULAR PATHOLOGY, BMJ PUBLISHING GROUP, LONDON, GB, vol. 54, no. 5, octobre 2001 (2001-10), pages 293-299, XP008010748 ISSN: 1366-8714
- C.G. LIN ET AL.: "CCN3 (NOV) is a novel angiogenic regulator of the CCN protein family." JOURNAL OF BIOLOGICAL CHEMISTRY, vol. 278, no. 26, 27 juin 2003 (2003-06-27), pages 24200-24208, XP002272895 BALTIMORE, MD, US cité dans la demande
- L.F. LAU ET AL.: "The CCN family of angiogenic regulators: the integrin connection." EXPERIMENTAL CELL RESEARCH, vol. 248, 1999, pages 44-57, XP002272896

### LISTE DE SEQUENCES

<110> CENTRE NATIONAL DE LA RECHERCHE SCIENTIFIQUE
<120> NOUVEL AGENT ANTI-ANGIOGENIQUE ET SON UTILISATION, NOTAMMENT DANS LE CADRE DU TRAITEMENT DES CANCERS
<130> WOB 03 AW CNR GIOG
<150> FR 03/09506
   <151> 2004-08-01
<160> 12
<170> PatentIn version 3.1
<210> 1
   <211> 2389
   <212> ADN
   <213> homo sapiens
<220>
   <221> CDS
   <222> (73)..(1143)
   <223>
<400> 1
   gggaaggcga gcagtgccaa tctacagcga agaaagtctc gtttggtaaa agcgagaggg      60
   gaaagcctga gc atg cag agt gtg cag agc acg agc ttt tgt ctc cga aag     111
                 Met Gln Ser Val Gln Ser Thr Ser Phe Cys Leu Arg Lys
                 1               5                   10
   cag tgc ctt tgc ctg acc ttc ctg ctt ctc cat ctc ctg gga cag gtc       159
   Gln Cys Leu Cys Leu Thr Phe Leu Leu Leu His Leu Leu Gly Gln Val
       15                  20                  25
   gct gcg act cag cgc tgc cct ccc cag tgc ccg ggc cgg tgc cct gcg       207
   Ala Ala Thr Gln Arg Cys Pro Pro Gln Cys Pro Gly Arg Cys Pro Ala
   30                  35                  40                  45
   acg ccg ccg acc tgc gcc ccc ggg gtg cgc gcg gtg ctg gac ggc tgc       255
   Thr Pro Pro Thr Cys Ala Pro Gly Val Arg Ala Val Leu Asp Gly Cys
                   50                  55                  60
   tca tgc tgt ctg gtg tgt gcc cgc cag cgt ggc gag agc tgc tca gat       303
   Ser Cys Cys Leu Val Cys Ala Arg Gln Arg Gly Glu Ser Cys Ser Asp
               65                  70                  75
   ctg gag cca tgc gac gag agc agt ggc ctc tac tgt gat cgc agc gcg       351
   Leu Glu Pro Cys Asp Glu Ser Ser Gly Leu Tyr Cys Asp Arg Ser Ala
           80                  85                  90
   gac ccc agc aac cag act ggc atc tgc acg gcg gta gag gga gat aac       399
   Asp Pro Ser Asn Gln Thr Gly Ile Cys Thr Ala Val Glu Gly Asp Asn
       95                  100                 105
   tgt gtg ttc gat ggg gtc atc tac cgc agt gga gag aaa ttt cag cca       447
   Cys Val Phe Asp Gly Val Ile Tyr Arg Ser Gly Glu Lys Phe Gln Pro
   110                 115                 120                 125
   agc tgc aaa ttc cag tgc acc tgc aga gat ggg cag att ggc tgt gtg      495
   Ser Cys Lys Phe Gln Cys Thr Cys Arg Asp Gly Gln Ile Gly Cys Val
                   130                 135                 140
   ccc cgc tgt cag ctg gat gtg cta ctg cct gag cct aac tgc cca gct      543
   Pro Arg Cys Gln Leu Asp Val Leu Leu Pro Glu Pro Asn Cys Pro Ala
               145                 150                 155
   cca aga aaa gtt gag gtg cct gga gag tgc tgt gaa aag tgg atc tgt      591
   Pro Arg Lys Val Glu Val Pro Gly Glu Cys Cys Glu Lys Trp Ile Cys
           160                 165                 170
   ggc cca gat gag gag gat tca ctg gga ggc ctt acc ctt gca gct tac      639
   Gly Pro Asp Glu Glu Asp Ser Leu Gly Gly Leu Thr Leu Ala Ala Tyr
       175                 180                 185
   agg cca gaa gcc acc cta gga gta gaa gtc tct gac tca agt gtc aac      687
   Arg Pro Glu Ala Thr Leu Gly Val Glu Val Ser Asp Ser Ser Val Asn
   190                 195                 200                 205
   tgc att gaa cag acc aca gag tgg aca gca tgc tcc aag agc tgt ggt      735
   Cys Ile Glu Gln Thr Thr Glu Trp Thr Ala Cys Ser Lys Ser Cys Gly
                   210                 215                 220
   atg ggg ttc tcc acc cgg gtc acc aat agg aac cgt caa tgt gag atg      783
   Met Gly Phe Ser Thr Arg Val Thr Asn Arg Asn Arg Gln Cys Glu Met
               225                 230                 235
   ctg aaa cag act cgg ctc tgc atg gtg cgg ccc tgt gaa caa gag cca      831
   Leu Lys Gln Thr Arg Leu Cys Met Val Arg Pro Cys Glu Gln Glu Pro
           240                 245                 250
   gag cag cca aca gat aag aaa gga aaa aag tgt ctc cgc acc aag aag      879
   Glu Gln Pro Thr Asp Lys Lys Gly Lys Lys Cys Leu Arg Thr Lys Lys
       255                 260                 265
   tca ctc aaa gcc atc cac ctg cag ttc aag aac tgc acc agc ctg cac      927
   Ser Leu Lys Ala Ile His Leu Gln Phe Lys Asn Cys Thr Ser Leu His
   270                 275                 280                 285
   acc tac aag ccc agg ttc tgt ggg gtc tgc agt gat ggc cgc tgc tgc      975
   Thr Tyr Lys Pro Arg Phe Cys Gly Val Cys Ser Asp Gly Arg Cys Cys
                   290                 295                 300
   act ccc cac aat acc aaa acc atc cag gca gag ttt cag tgc tcc cca     1023
   Thr Pro His Asn Thr Lys Thr Ile Gln Ala Glu Phe Gln Cys Ser Pro
               305                 310                 315
   ggg caa ata gtc aag aag cca gtg atg gtc att ggg acc tgc acc tgt     1071
   Gly Gln Ile Val Lys Lys Pro Val Met Val Ile Gly Thr Cys Thr Cys
           320                 325                 330
   cac acc aac tgt cct aag aac aat gag gcc ttc ctc cag gag ctg gag     1119
   His Thr Asn Cys Pro Lys Asn Asn Glu Ala Phe Leu Gln Glu Leu Glu
       335                 340                 345
   ctg aag act acc aga ggg aaa atg taacctatca ctcaagaagc acacctacag    1173
   Leu Lys Thr Thr Arg Gly Lys Met
   350                 355
   agcacctgta gctgctgcgc cacccaccat caaaggaata taagaaaagt aatgaagaat   1233
   cacgatttca tccttgaatc ctatgtattt tcctaatgtg atcatatgag gacctttcat   1293
   atctgtcttt tatttaacaa aaaatgtaat taactgtaaa cttggaatca aggtaagctc   1353
   aggatatggc ttaggaatga cttactttcc tgtggtttta ttacaaatgc aaatttctat   1413
   aaatttaaga aaacaagtat ataatttact ttgtagactg tttcacattg cactcatcat   1473
   attttgttgt gcactagtgc aattccaaga aaatatcact gtaatgagtc agtgaagtct   1533
   agaatcatac ttaacatttc attgtacaag tattacaacc atatattgag gttcattggg   1593
   aagattctct attggctccc tttttgggta aaccagctct gaacttccaa gctccaaatc   1653
   caaggaaaca tgcagctctt caacatgaca tccagagatg actattactt ttctgtttag   1713
   ttttacacta ggaaacgtgt tgtatctaca gtaatgaaat gtttactaag tggactggtg   1773
   tcataaactt tctccattta agacacattg actcctttcc aatagaaaga aactaaacag   1833
   aaaactccca atacaaagat gactggtccc tcatagccct cagacattta tatattggaa   1893
   gctgctgagg cccccaagtt ttttaattaa gcagaaacag catattagca gggattctct   1953
   catctaactg atgagtaaac tgaggcccaa agcacttgct tacatcctct gatagctgtt   2013
   tcaaatgtgc attttgtgga attttgagaa aaatagagca aaatcaacat gactggtggt   2073
   gagagaccac acattttatg agagtttgga attattgtag acatgcccaa aacttatcct   2133
   tgggccataa ttatgaaaac tcatgatcaa gatatatgtg tatacataca tgtatctggt   2193
   ttgtcaggct acaaggtagg ctgcaaaatt aaatctagac attcttttaa tgccaccaca   2253
   cgtgttccgc ttctctcttt taaagtattt ataaaaatat aaattgtaca ttttgtaaaa   2313
   tattatgttt gatttctcta cttgtcatat cactaaataa acacgatttt attgctgaaa   2373
   aaaaaaaaaa aaaaaa                                                   2389
<210> 2
   <211> 357
   <212> PRT
   <213> homo sapiens
<400> 2
   Met Gln Ser Val Gln Ser Thr Ser Phe Cys Leu Arg Lys Gln Cys Leu
   1               5                   10                  15
   Cys Leu Thr Phe Leu Leu Leu His Leu Leu Gly Gln Val Ala Ala Thr
               20                  25                  30
   Gln Arg Cys Pro Pro Gln Cys Pro Gly Arg Cys Pro Ala Thr Pro Pro
           35                  40                  45
   Thr Cys Ala Pro Gly Val Arg Ala Val Leu Asp Gly Cys Ser Cys Cys
       50                  55                  60
   Leu Val Cys Ala Arg Gln Arg Gly Glu Ser Cys Ser Asp Leu Glu Pro
   65                  70                  75                  80
   Cys Asp Glu Ser Ser Gly Leu Tyr Cys Asp Arg Ser Ala Asp Pro Ser
                   85                  90                  95
   Asn Gln Thr Gly Ile Cys Thr Ala Val Glu Gly Asp Asn Cys Val Phe
               100                 105                 110
   Asp Gly Val Ile Tyr Arg Ser Gly Glu Lys Phe Gln Pro Ser Cys Lys
           115                 120                 125
   Phe Gln Cys Thr Cys Arg Asp Gly Gln Ile Gly Cys Val Pro Arg Cys
       130                 135                 140
   Gln Leu Asp Val Leu Leu Pro Glu Pro Asn Cys Pro Ala Pro Arg Lys
   145                 150                 155                 160
   Val Glu Val Pro Gly Glu Cys Cys Glu Lys Trp Ile Cys Gly Pro Asp
                   165                 170                 175
   Glu Glu Asp Ser Leu Gly Gly Leu Thr Leu Ala Ala Tyr Arg Pro Glu
               180                 185                 190
   Ala Thr Leu Gly Val Glu Val Ser Asp Ser Ser Val Asn Cys Ile Glu
           195                 200                 205
   Gln Thr Thr Glu Trp Thr Ala Cys Ser Lys Ser Cys Gly Met Gly Phe
       210                 215                 220
   Ser Thr Arg Val Thr Asn Arg Asn Arg Gln Cys Glu Met Leu Lys Gln
   225                 230                 235                 240
   Thr Arg Leu Cys Met Val Arg Pro Cys Glu Gln Glu Pro Glu Gln Pro
                   245                 250                 255
   Thr Asp Lys Lys Gly Lys Lys Cys Leu Arg Thr Lys Lys Ser Leu Lys
               260                 265                 270
   Ala Ile His Leu Gln Phe Lys Asn Cys Thr Ser Leu His Thr Tyr Lys
           275                 280                 285
   Pro Arg Phe Cys Gly Val Cys Ser Asp Gly Arg Cys Cys Thr Pro His
       290                 295                 300
   Asn Thr Lys Thr Ile Gln Ala Glu Phe Gln Cys Ser Pro Gly Gln Ile
   305                 310                 315                 320
   Val Lys Lys Pro Val Met Val Ile Gly Thr Cys Thr Cys His Thr Asn
                   325                 330                 335
   Cys Pro Lys Asn Asn Glu Ala Phe Leu Gln Glu Leu Glu Leu Lys Thr
               340                 345                 350
   Thr Arg Gly Lys Met
           355
<210> 3
   <211> 216
   <212> ADN
   <213> séquence artificielle
<220>
   <223> fragment de la protéine NOV
<220>
   <221> CDS
   <222> (1)..(216)
   <223>
<400> 3
   cag cgc tgc cct ccc cag tgc ccg ggc cgg tgc cct gcg acg ccg ccg      48
   Gln Arg Cys Pro Pro Gln Cys Pro Gly Arg Cys Pro Ala Thr Pro Pro
   1               5                   10                  15
   acc tgc gcc ccc ggg gtg cgc gcg gtg ctg gac ggc tgc tca tgc tgt      96
   Thr Cys Ala Pro Gly Val Arg Ala Val Leu Asp Gly Cys Ser Cys Cys
               20                  25                  30
   ctg gtg tgt gcc cgc cag cgt ggc gag agc tgc tca gat ctg gag cca     144
   Leu Val Cys Ala Arg Gln Arg Gly Glu Ser Cys Ser Asp Leu Glu Pro
           35                  40                  45
   tgc gac gag agc agt ggc ctc tac tgt gat cgc agc gcg gac ccc agc     192
   Cys Asp Glu Ser Ser Gly Leu Tyr Cys Asp Arg Ser Ala Asp Pro Ser
       50                  55                  60
   aac cag act ggc atc tgc acg gcg                                     216
   Asn Gln Thr Gly Ile Cys Thr Ala
   65                  70
<210> 4
   <211> 72
   <212> PRT
   <213> séquence artificielle
<220>
   <223> fragment de la protéine NOV
<400> 4
   Gln Arg Cys Pro Pro Gln Cys Pro Gly Arg Cys Pro Ala Thr Pro Pro
   1               5                   10                  15
   Thr Cys Ala Pro Gly Val Arg Ala Val Leu Asp Gly Cys Ser Cys Cys
               20                  25                  30
   Leu Val Cys Ala Arg Gln Arg Gly Glu Ser Cys Ser Asp Leu Glu Pro
           35                  40                  45
   Cys Asp Glu Ser Ser Gly Leu Tyr Cys Asp Arg Ser Ala Asp Pro Ser
       50                  55                  60
   Asn Gln Thr Gly Ile Cys Thr Ala
   65                  70
<210> 5
   <211> 201
   <212> ADN
   <213> séquence artificielle
<220>
   <223> fragment de la protéine NOV
<220>
   <221> CDS
   <222> (1)..(201)
   <223>
<400> 5
   gat aac tgt gtg ttc gat ggg gtc atc tac cgc agt gga gag aaa ttt       48
   Asp Asn Cys Val Phe Asp Gly Val Ile Tyr Arg Ser Gly Glu Lys Phe
   1               5                   10                  15
   cag cca agc tgc aaa ttc cag tgc acc tgc aga gat ggg cag att ggc       96
   Gln Pro Ser Cys Lys Phe Gln Cys Thr Cys Arg Asp Gly Gln Ile Gly
               20                  25                  30
   tgt gtg ccc cgc tgt cag ctg gat gtg cta ctg cct gag cct aac tgc      144
   Cys Val Pro Arg Cys Gln Leu Asp Val Leu Leu Pro Glu Pro Asn Cys
           35                  40                  45
   cca gct cca aga aaa gtt gag gtg cct gga gag tgc tgt gaa aag tgg      192
   Pro Ala Pro Arg Lys Val Glu Val Pro Gly Glu Cys Cys Glu Lys Trp
       50                  55                  60
   atc tgt ggc                                                          201
   Ile Cys Gly
   65
<210> 6
   <211> 67
   <212> PRT
   <213> séquence artificielle
<220>
   <223> fragment de la protéine NOV
<400> 6
   Asp Asn Cys Val Phe Asp Gly Val Ile Tyr Arg Ser Gly Glu Lys Phe
   1               5                   10                  15
   Gln Pro Ser Cys Lys Phe Gln Cys Thr Cys Arg Asp Gly Gln Ile Gly
               20                  25                  30
   Cys Val Pro Arg Cys Gln Leu Asp Val Leu Leu Pro Glu Pro Asn Cys
           35                  40                  45
   Pro Ala Pro Arg Lys Val Glu Val Pro Gly Glu Cys Cys Glu Lys Trp
       50                  55                  60
   Ile Cys Gly
   65
<210> 7
   <211> 135
   <212> ADN
   <213> séquence artificielle
<220>
   <223> fragment de la protéine NOV
<220>
   <221> CDS
   <222> (1)..(135)
   <223>
<400> 7
   tgc att gaa cag acc aca gag tgg aca gca tgc tcc aag agc tgt ggt       48
   Cys Ile Glu Gln Thr Thr Glu Trp Thr Ala Cys Ser Lys Ser Cys Gly
   1               5                   10                  15
   atg ggg ttc tcc acc cgg gtc acc aat agg aac cgt caa tgt gag atg       96
   Met Gly Phe Ser Thr Arg Val Thr Asn Arg Asn Arg Gln Cys Glu Met
               20                  25                  30
   ctg aaa cag act cgg ctc tgc atg gtg cgg ccc tgt gaa                   135
   Leu Lys Gln Thr Arg Leu Cys Met Val Arg Pro Cys Glu
           35                  40                  45
<210> 8
   <211> 45
   <212> PRT
   <213> séquence artificielle
<220>
   <223> fragment de la protéine NOV
<400> 8
   Cys Ile Glu Gln Thr Thr Glu Trp Thr Ala Cys Ser Lys Ser Cys Gly
   1               5                   10                  15
   Met Gly Phe Ser Thr Arg Val Thr Asn Arg Asn Arg Gln Cys Glu Met
               20                  25                  30
   Leu Lys Gln Thr Arg Leu Cys Met Val Arg Pro Cys Glu
           35                  40                  45
<210> 9
   <211> 225
   <212> ADN
   <213> séquence artificielle
<220>
   <223> fragment de la protéine NOV
<220>
   <221> CDS
   <222> (1)..(225)
   <223>
<400> 9
   tgt ctc cgc acc aag aag tca ctc aaa gcc atc cac ctg cag ttc aag      48
   Cys Leu Arg Thr Lys Lys Ser Leu Lys Ala Ile His Leu Gln Phe Lys
   1               5                   10                  15
   aac tgc acc agc ctg cac acc tac aag ccc agg ttc tgt ggg gtc tgc      96
   Asn Cys Thr Ser Leu His Thr Tyr Lys Pro Arg Phe Cys Gly Val Cys
               20                  25                  30
   agt gat ggc cgc tgc tgc act ccc cac aat acc aaa acc atc cag gca     144
   Ser Asp Gly Arg Cys Cys Thr Pro His Asn Thr Lys Thr Ile Gln Ala
           35                  40                  45
   gag ttt cag tgc tcc cca ggg caa ata gtc aag aag cca gtg atg gtc     192
   Glu Phe Gln Cys Ser Pro Gly Gln Ile Val Lys Lys Pro Val Met Val
       50                  55                  60
   att ggg acc tgc acc tgt cac acc aac tgt cct                          225
   Ile Gly Thr Cys Thr Cys His Thr Asn Cys Pro
   65                  70                  75
<210> 10
   <211> 75
   <212> PRT
   <213> séquence artificielle
<220>
   <223> fragment de la protéine NOV
<400> 10
   Cys Leu Arg Thr Lys Lys Ser Leu Lys Ala Ile His Leu Gln Phe Lys
   1               5                   10                  15
   Asn Cys Thr Ser Leu His Thr Tyr Lys Pro Arg Phe Cys Gly Val Cys
               20                  25                  30
   Ser Asp Gly Arg Cys Cys Thr Pro His Asn Thr Lys Thr Ile Gln Ala
           35                  40                  45
   Glu Phe Gln Cys Ser Pro Gly Gln Ile Val Lys Lys Pro Val Met Val
       50                  55                  60
   Ile Gly Thr Cys Thr Cys His Thr Asn Cys Pro
   65                  70                  75
<210> 11
   <211> 510
   <212> ADN
   <213> Homo sapiens
<220>
   <221> CDS
   <222> (1)..(510)
   <223>
<400> 11
   gct tac agg cca gaa gcc acc cta gga gta gaa gtc tct gac tca agt      48
   Ala Tyr Arg Pro Glu Ala Thr Leu Gly Val Glu Val Ser Asp Ser Ser
   1               5                   10                  15
   gtc aac tgc att gaa cag acc aca gag tgg aca gca tgc tcc aag agc      96
   Val Asn Cys Ile Glu Gln Thr Thr Glu Trp Thr Ala Cys Ser Lys Ser
               20                  25                  30
   tgt ggt atg ggg ttc tcc acc cgg gtc acc aat agg aac cgt caa tgt     144
   Cys Gly Met Gly Phe Ser Thr Arg Val Thr Asn Arg Asn Arg Gln Cys
           35                  40                  45
   gag atg ctg aaa cag act cgg ctc tgc atg gtg cgg ccc tgt gaa caa     192
   Glu Met Leu Lys Gln Thr Arg Leu Cys Met Val Arg Pro Cys Glu Gln
       50                  55                  60
   gag cca gag cag cca aca gat aag aaa gga aaa aag tgt ctc cgc acc     240
   Glu Pro Glu Gln Pro Thr Asp Lys Lys Gly Lys Lys Cys Leu Arg Thr
   65                  70                  75                  80
   aag aag tca ctc aaa gcc atc cac ctg cag ttc aag aac tgc acc agc     288
   Lys Lys Ser Leu Lys Ala Ile His Leu Gln Phe Lys Asn Cys Thr Ser
                   85                  90                  95
   ctg cac acc tac aag ccc agg ttc tgt ggg gtc tgc agt gat ggc cgc     336
   Leu His Thr Tyr Lys Pro Arg Phe Cys Gly Val Cys Ser Asp Gly Arg
               100                 105                 110
   tgc tgc act ccc cac aat acc aaa acc atc cag gca gag ttt cag tgc     384
   Cys Cys Thr Pro His Asn Thr Lys Thr Ile Gln Ala Glu Phe Gln Cys
           115                 120                 125
   tcc cca ggg caa ata gtc aag aag cca gtg atg gtc att ggg acc tgc     432
   Ser Pro Gly Gln Ile Val Lys Lys Pro Val Met Val Ile Gly Thr Cys
       130                 135                 140
   acc tgt cac acc aac tgt cct aag aac aat gag gcc ttc ctc cag gag     480
   Thr Cys His Thr Asn Cys Pro Lys Asn Asn Glu Ala Phe Leu Gln Glu
   145                 150                 155                 160
   ctg gag ctg aag act acc aga ggg aaa atg                             510
   Leu Glu Leu Lys Thr Thr Arg Gly Lys Met
                   165                 170
<210> 12
   <211> 170
   <212> PRT
   <213> Homo sapiens
<400> 12
   Ala Tyr Arg Pro Glu Ala Thr Leu Gly Val Glu Val Ser Asp Ser Ser
   1               5                   10                  15
   Val Asn Cys Ile Glu Gln Thr Thr Glu Trp Thr Ala Cys Ser Lys Ser
               20                  25                  30
   Cys Gly Met Gly Phe Ser Thr Arg Val Thr Asn Arg Asn Arg Gln Cys
           35                  40                  45
   Glu Met Leu Lys Gln Thr Arg Leu Cys Met Val Arg Pro Cys Glu Gln
       50                  55                  60
   Glu Pro Glu Gln Pro Thr Asp Lys Lys Gly Lys Lys Cys Leu Arg Thr
   65                  70                  75                  80
   Lys Lys Ser Leu Lys Ala Ile His Leu Gln Phe Lys Asn Cys Thr Ser
                   85                  90                  95
   Leu His Thr Tyr Lys Pro Arg Phe Cys Gly Val Cys Ser Asp Gly Arg
               100                 105                 110
   Cys Cys Thr Pro His Asn Thr Lys Thr Ile Gln Ala Glu Phe Gln Cys
           115                 120                 125
   Ser Pro Gly Gln Ile Val Lys Lys Pro Val Met Val Ile Gly Thr Cys
       130                 135                 140
   Thr Cys His Thr Asn Cys Pro Lys Asn Asn Glu Ala Phe Leu Gln Glu
   145                 150                 155                 160
   Leu Glu Leu Lys Thr Thr Arg Gly Lys Met
                   165                 170

## Description

La présente invention a pour objet un nouvel agent anti-angiogénique, ainsi que son utilisation, notamment dans le cadre du traitement des cancers.

L'angiogenèse est un processus de croissance de nouveaux capillaires sanguins à partir de vaisseaux préexistants. Trois phénomènes particuliers sont notamment à la base de ce processus : la prolifération, la migration et la différenciation (la tubulogenèse) des cellules endothéliales. L'angiogenèse est activée par certains facteurs de croissances, dits facteurs angiogéniques, tels que le VEGF (*Vascular Endothelial Growth Factor*, facteur de croissance endothélial vasculaire), le FGF-1 (*Fibroblast Growth Factor 1*, facteur de croissance des fibroblastes 1) ou le FGF-2 (*Fibroblast Growth Factor 2*, facteur de croissance des fibroblastes 2).

En temps normal, l'angiogenèse est essentiellement restreinte au système reproducteur femelle et à la cicatrisation des plaies. Cependant, l'angiogenèse est également impliquée dans de nombreux cas pathologiques, tels que la rétinopathie diabétique, le psoriasis, la polyarthrite rhumatoïde, la dégénérescence maculaire liée à l'âge, et les cancers. En effet, dans ce dernier cas il a été montré que la croissance tumorale était grandement favorisée par l'apparition au sein de ces tumeurs d'une néo-vascularisation résultant en particulier de la sécrétion par les tumeurs de facteurs angiogéniques.

De nombreuses tentatives de traitements thérapeutiques basées sur l'utilisation de protéines anti-angiogéniques sont en cours. Parmi ces composés, l'un des plus prometteur est l'endostatine (O'Reilly et al., 1997), qui est actuellement, en essais cliniques de phase I (Herbst et al., 2002). L'endostatine est une protéine de 20 kDa correspondant à un fragment du collagène XVIII. Le mécanisme d'action de l'endostatine reste inconnu.

Certaines tentatives thérapeutiques reposent sur l'élucidation de mécanismes d'action connus. Ainsi les cellules endothéliales en prolifération expriment l'intégrine avb3 alors que les cellules endothéliales quiescentes ne l'expriment pas (Brooks, 1994). Cette observation a permis de mettre au point des inhibiteurs de cette molécule actuellement en cours d'essais cliniques.

Parmi tous les acteurs moléculaires impliqués dans l'activation de l'angiogenèse, seul le VEGF a fait la preuve de son efficacité dans pratiquement tous les modèles expérimentaux de mesure d'activité de l'angiogenèse (Ortéga, 1999). De plus, au printemps 2003, il a été rendu public par la société Genentech que des anticorps anti-VEGF exerçaient une activité anti-tumorale chez les malades atteints de cancer du colon. Ainsi donc il est de toute première importance de rechercher des molécules pouvant se lier au VEGF et par là même d'exercer une activité anti-tumorale comparable à celle des anticorps anti-VEGF.

Le gène nov, tout d'abord identifié dans des néphroblastomes aviaires (Joliot et al., 1992 ; Martinerie et Perbal, 1991), a été cloné chez l'homme (*nov*H)(Martinerie et al., 1994), la souris (*nov*M)(Snaith et al., 1996) et *Xenopus laevis* (Ying et Ling, 1996). La protéine NOV, codée par le gène nov, dont la fonction est à ce jour inconnue, appartient à la famille CCN (Bork, 1993) qui comprend les protéines suivantes : CYR61 (Lau et Nathans, 1985), CTGF (Bradham et al., 1991), ELM-1 ou WISP-1 (Pennica et al., 1998 ; Hashimoto et al., 1998), R-COP ou WISP-2 (Pennica et al., 1998 ; Kumar et al., 1999 ; Brigstock, 1999) et WISP-3 (Pennica et al., 1998). Ces protéines sont toutes constituées de quatre domaines distincts : une protéine se liant à un facteur de croissance analogue à l'insuline (IGFBP), un domaine de répétition facteur Willebrand de type C, un domaine de répétition thrombospondine de type I et un domaine COOH-terminal. Les protéines de la famille CCN régulent différents procédés cellulaires normaux comprenant la prolifération, l'adhésion, l'apoptose et la chimiotaxie. Elles sont également impliquées dans l'implantation, la formation squelettique, le développement embryonnaire et dans différentes maladies telles que la fibrose, la cicatrisation et les cancers (Chevalier et al., 1998).

La protéine NOV humaine (NOVH) peut être détectée dans les tissus normaux (reins, muscles, cartilage, cerveau, poumons, ovaires, coeur et corticosurrénale) à différents niveaux (Joliot et al., 1992 ; Martinerie et al., 2001 ; Kocialkowski et al., 2001 ; Perbal et al., 1999) et son expression varie au cours du développement.

A ce jour les fonctions exercées par la protéine NOV ne sont pas clairement établies. Il a été récemment proposé que NOV pourrait exercer une action proangiogénique (Lin, 2003) en permettant de se lier à certaines intégrines (avb3, a6b1 et a5b1). De plus ces auteurs montrent que NOV exerce une activité proangiogénique dans le modèle de cornée de lapin. Cependant, il a été démontré que cet essai peut conduire à des résultats faussement positifs par relargage de facteurs angiogéniques synthétisés et stockés dans la cornée (Plouët, 1997).

Ainsi la présente invention résulte de la mise en évidence de l'activité anti-angiogénique de NOV du fait de sa liaison à VEGF.

La présente invention a pour but de fournir un nouvel agent anti-angiogénique ayant un nouveau mécanisme d'action.

La présente invention repose sur la constatation faite par les Inventeurs que l'utilisation :
- d'une protéine caractérisée en ce qu'elle comprend ou est constituée par :
   * la protéine NOV, représentée par la séquence SEQ ID NO : 2, ou
   * un fragment de cette protéine, sous réserve que ce fragment présente une activité d'inhibition de l'angiogenèse, ledit fragment comprenant notamment d'environ 40 à environ 180 acides aminés, et étant notamment représenté par l'une des séquences suivantes SEQ NO : 4, SEQ ID NO : 6, SEQ ID NO : 8, SEQ ID NO : 10 ou SEQ ID NO 12, ou
   * toute séquence dérivée de la séquence SEQ ID NO : 2 ou d'un fragment défini ci-dessus, notamment par substitution, suppression ou addition d'un ou plusieurs acides aminés, sous réserve que cette séquence dérivée présente une activité d'inhibition de l'angiogenèse, ou
   * toute séquence homologue de la séquence SEQ ID NO : 2 ou d'un fragment défini ci-dessus, ayant de référence une homologie d'au moins environ 80%, et notamment 85%, avec la région comprise entre les acides aminés en position (33) et (338) de la séquence SEQ ID NO : 2, sous réserve que cette séquence homologue présente une activité d'inhibition de l'angiogenèse,
- d'une séquence nucléotidique caractérisée en ce qu'elle comprend ou est constituée par une séquence nucléotidique codant :
   * soit pour la protéine NOV telle que définie ci-dessus,
   * soit pour un fragment de la protéine NOV tel que défini ci-dessus,
   * soit pour une séquence dérivée de la.protéine NOV telle que définie ci-dessus,
   * soit pour une séquence homologue de la protéine NOV telle que définie ci-dessus,
ladite séquence nucléotidique correspondant notamment à la séquence nucléotidique SEQ ID NO : 1 codant pour SEQ ID NO : 2, ou à la séquence SEQ ID NO : 3 codant pour SEQ ID NO : 4, ou à la séquence SEQ ID NO : 5 codant pour SEQ ID NO : 6, ou à la séquence SEQ ID NO : 7 codant pour SEQ ID NO : 8, ou à la séquence SEQ ID NO : 9 codant pour SEQ ID NO : 10, ou à la séquence SEQ ID NO : 11 codant pour SEQ ID NO : 12,
- d'un anticorps anti-idiotypique de la protéine NOV, peut être envisagée pour la préparation d'un médicament destiné au traitement :
- de pathologies nécessitant l'inhibition de la prolifération endothéliale, notamment dans le cadre des pathologies suivantes : la dégénérescence maculaire liée à l'âge; les rétinopathies diabétiques, la polyarthrite rhumatoïde, les angiomes, les angiosarcomes, en particulier la maladie de Castelman et le sarcome de Kaposi, ou
- de pathologies nécessitant l'inhibition de l'activation endothéliale, notamment dans le cadre des pathologies suivantes : le rejet d'allogreffe et de xénogreffe, les acrocyanoses, les sclérodermies, ou dans le cadre de la préparation de greffons entre le prélèvement et la transplantation.

Aussi, l'invention concerne l'utilisation :
- d'une protéine caractérisée en ce qu'elle est constituée par :
   * la protéine NOV, représentée par la séquence SEQ ID NO : 2, ou
   * un fragment de cette protéine, représenté par la séquence SEQ ID NO : 12
- d'une séquence nucléotidigue caractérisée en ce qu'elle comprend ou est constituée par une séquence nucléotidique codant :
   * soit pour la protéine NOV telle que définie ci-dessus,
   * soit pour un fragment de la protéine NOV tel que défini ci-dessus,
   ladite séquence nucléotidique correspondant à la séquence nucléotidique SEQ ID NO : 1 codant pour SEQ ID NO : 2, ou à la séquence SEQ ID NO : 11 codant pour SEQ ID NO : 12,
- d'un anticorps anti-idiotyque de la protéine NOV représentée par la séquence SEQ ID NO : 2, ledit anticorps mimant les fonctions de la protéine NOV en reconnaissant le VEGF,
pour la préparation d'un médicament destiné au traitement :
■ de pathologies nécessitant l'inhibition de la prolifération endothéliale, dans le cadre des pathologies suivantes : la dégénérescence maculaire liée à l'âge, les rétinopathies diabétiques, la polyarthrite rhumatoïde, les angiomes, les angiosarcomes, en particulier la maladie de Castelman et le sarcome de Kaposi, ou
■ de pathologies nécessitant l'inhibition de l'activation endothéliale, dans le cadre des pathologies suivantes : le rejet d'allogreffe et de xénogreffe, les acrocyanoses, les sclérodermies, ou dans le cadre de la préparation de greffons entre le prélèvement et la transplantation.

Par ailleurs, la présente invention repose sur la constatation précédente que l'utilisation d'une protéine caractérisée en ce qu'elle comprend ou est constituée par :
* la protéine NOV, représentée parle séquence SEQ ID NO: 2, ou
* un fragment de cette protéine, sous réserve que ce fragment présente une activité d'inhibition de l'angiogenèse, ledit fragment comprenant notamment d'environ 40 à environ 180 acides aminés, et de préférence d'environ 40 à environ 80 acides aminés, et étant notamment représenté par l'une des séquences suivantes SEQ ID NO : 4, SEQ ID NO : 6, SEQ ID NO : 8, SEQ ID NO : 10 ou SEQ ID NO : 12, ou
* toute séquence dérivée de la séquence SEQ ID NO : 2 ou d'un fragment défini ci-dessus, notamment par substitution, suppression ou addition d'un ou plusieurs acides aminés, sous réserve que cette séquence dérivée présente une activité d'inhibition de l'angiogenèse, ou
* toute séquence homologue de la séquence SEQ ID NO : 2 ou d'un fragment défini ci-dessus, ayant de préférence une homologie d'au moins environ 80%, et notamment 85%, avec la région comprise entre les acides aminés en position (33) et (338) de la séquence SEQ ID NO : 2, sous réserve que cette séquence homologue présente une activité d'inhibition de l'angiogenèse, peut être
envisagée pour la préparation d'un médicament destiné au traitement :
- de pathologies nécessitant l'inhibition de la prolifération endothéliale, notamment dans le cadre des pathologies suivantes : la dégénérescence maculaire liée à l'âge, les rétinopathies diabétiques, la polyarthrite rhumatoïde, les angiomes, les angiosarcomes, en particulier la maladie de Castelman et le sarcome de Kaposi, ou
- de pathologies nécessitant l'inhibition de l'activation endothéliale, notamment dans le cadre des pathologies suivantes : le rejet d'allogreffe et de xénogreffe, les acrocyanoses, les sclérodermies, ou dans le cadre de la préparation de greffons entre le prélèvement et la transplantation.

La séquence SEQ ID NO : 2 correspond à la protéine humaine NOV codée par la séquence nucléotidique SEQ ID NO : 1.

La séquence SEQ ID NO : 4 correspond au fragment IGFBP (protéine se liant à un facteur de croissance analogue à l'insuline) de la protéine humaine NOV, ledit fragment étant codé par la séquence nucléotidique SEQ ID NO : 3. Ce fragment comprend 72 acides aminés et correspond au fragment de la protéine NOV allant du résidu 33 au résidu 104 de la séquence SEQ ID NO : 2.

La séquence SEQ ID NO : 6 correspond au fragment VWC (domaine de répétition facteur Willebrand de type C) de la protéine humaine NOV, ledit fragment étant codé par la séquence nucléotidique SEQ ID NO : S. Ce fragment comprend 67 acides aminés et correspond au fragment de la protéine NOV allant du résidu 108 au résidu 174 de la séquence SEQ ID NO : 2.

La séquence SEQ ID NO: 8 correspond au fragment TSP-1 (domaine de répétition thrombospondine de type I) de la protéine humaine NOV, ledit fragment étant codé par la séquence nucléotidique SEQ ID NO : 7. Ce fragment comprend 45 acides aminés et correspond au fragment de la protéine NOV allant du résidu 206 au résidu 250 de la séquence SEQ ID NO : 2.

La séquence SEQ ID NO : 10 correspond au fragment CT (domaine COOH-terminal) de la protéine humaine NOV, ledit fragment étant codé par la séquence nucléotidique SEQ ID NO : 9. Ce fragment comprend 75 acides aminés et correspond au fragment de la protéine NOV allant du résidu 264 au résidu 338 de la séquence SEQ ID NO : 2.

La séquence SEQ ID NO : 12 correspond au fragment C-terminal de la protéine humaine NOV, ledit fragment étant codé par la séquence nucléotidique SEQ ID NO : 11. Ce fragment comprend 170 acides aminés et correspond au fragment de la protéine NOV allant du résidu 188 au résidu 357 de la séquence SEQ ID NO : 2.

L'activité d'inhibition de l'angiogenèse est également désignée activité anti-angiogénique. Cette activité peut être par exemple mise en évidence *in vitro* en démontrant l'inhibition, à la fois de la multiplication, de la migration et de la différenciation, de cellules endothéliales par les séquences peptidique de l'invention. La mesure de l'inhibition de la multiplication des cellules endothéliales peut être réalisée en cultivant des cellules endothéliales en présence de la séquence peptidique dont on souhaite évaluer l'activité. La mesure de l'inhibition de la migration des cellules endothéliales peut être réalisée en effectuant une « blessure » sur un tapis de cellules endothéliales et en incubant ensuite les cellules en présence de la séquence peptidique à tester. On mesure alors le nombre de cellules ayant migré sur la blessure. La mesure de l'inhibition de la différenciation (tubulogenèse) des cellules endothéliales peut être réalisée en mesurant la longueur de tubules formés par des cellules endothéliales cultivées sur gel en présence de la séquence peptidique à tester.

Parmi les modèles classiques de mesure d'angiogenèse, on peut citer les modèles par délivrance locale comme :
- l'injection sous-cutanée de Matrigel (Becton Dickinson) imprégné du composé de l'invention (Inoki et al., 2002), ou
- le dépôt sur la membrane chorio-allantoïdienne de poulet d'un implant contenant un composé de l'invention (Celerier et al., 2002).

Le composé de l'invention peut être injecté par voie systémique (intraveineuse, intrapéritonéale, sous-cutanée) à des animaux chez qui on a créé une maladie angiogénique expérimentale. Le composé de l'invention peut aussi être injecté directement dans une tumeur. Alternativement la protéine NOV ou les fragments ou les anticorps anti-idiotypiques selon l'invention (décrits ci-après) peuvent être délivrés par une méthode de thérapie génique par voie locale ou systémique par toute méthode permettant l'expression de la protéine ou des fragments ou des anticorps anti-idiotypiques selon l'invention (virus ou plasmide contenant la séquence de NOV). Alternativement la séquence de NOV ou des fragments ou des anticorps anti-idiotypiques selon l'invention peut être insérée dans un plasmide qui est transfecté dans les cellules cancéreuses (ici la mesure consiste à mesurer l'évolution de tumeurs développées à partir de cellules cancéreuses transfectées par un plasmide contenant ou non la séquence de NOV ou d'un fragment). Tous ces procédés de mesure sont notamment décrits dans l'article de Jain et al. (1997).

On désigne par activité anti-tumorale, une activité permettant d'inhiber la croissance tumorale et/ou d'induire la régression voire la disparition de tumeurs. Cette activité peut être par exemple mise en évidence *in vivo* en mesurant la masse de tumeurs, dont on a induit le développement chez la souris par injection de cellules tumorales, en présence et en absence d'administration de séquences peptidiques de l'invention et/ou d'acides nucléiques exprimant les séquences peptidiques de l'invention.

L'expression "inhibition de la prolifération endothéliale" désigne toute substance capable de freiner la prolifération de cellules endothéliales selon le test décrit plus loin (partie expérimentale).

L'expression "activation endothéliale" correspond à toute pathologie impliquant des cellules endothéliales soumises à une concentration accrue en VEGF par rapport à l'état non pathologique.

Selon un mode de réalisation avantageux, la présente invention concerne l'utilisation telle que définie ci-dessus d'une protéine caractérisée en ce qu'elle comprend ou est constituée par la protéine NOV, représentée par la séquence SEQ ID NO: 2.

Une utilisation avantageuse selon la présente invention consiste en l'utilisation telle que définie ci-dessus d'une protéine caractérisée en ce qu'elle comprend ou est constituée par la protéine NOV, représentée par la séquence SEQ ID NO : 2, pour la préparation d'un médicament destiné au traitement de pathologies nécessitant l'inhibition de la prolifération endothéliale, notamment dans le cadre des pathologies suivantes : la dégénérescence maculaire liée à l'âge, les rétinopathies diabétiques, la polyarthrite rhumatoïde, les angiomes, les angiosarcomes, en particulier la maladie de Castelman et le sarcome de Kaposi.

Une utilisation avantageuse selon la présente invention consiste en l'utilisation telle que définie ci-dessus d'une protéine, caractérisée en ce qu'elle comprend ou est constituée par la protéine NOV, représentée par la séquence SEQ ID NO : 2, pour la préparation d'un médicament destiné au traitement de pathologies nécessitant l'inhibition de l'activation endothéliale, notamment dans le cadre des pathologies suivantes le rejet d'allogreffe et de xénogreffe, les acrocyanoses, les sclérodermies, ou dans le cadre de la préparation de greffons entre le prélèvement et la transplantation.

La présente invention repose également sur la constatation faite par les Inventeurs concernant une protéine caractérisée en ce qu'elle comprend ou est constituée par :
* un fragment de la protéine NOV, représentée par la séquence SEQ ID NO: 2, sous réserve que ce fragment présente une activité d'inhibition de l'angiogenèse, ledit fragment comprenant notamment d'environ 40 à environ 180 acides aminés, et étant notamment représenté par l'une des séquences suivantes SEQ ID NO : 4, SEQ ID NO: 6, SEQ ID NO : 8, SEQ ID NO : 10 ou SEQ ID NO : 12, ou
* toute séquence dérivée de la séquence SEQ ID NO: 2 ou d'un fragment défini ci-dessus, notamment par substitution, suppression ou addition d'un ou plusieurs acides aminés, sous réserve que cette séquence dérivée présente une activité d'inhibition de l'angiogenèse, ou
* toute séquence homologue de la séquence SEQ ID NO : 2. ou d'un fragment défini ci-dessus, ayant de préférence une homologie d'au moins environ 80%, et notamment 85%, avec la région comprise entre les acides aminés en position (33) et (338) de la séquence SEQ ID NO: 2, sous réserve que cette séquence homologue présente une activité d'inhibition de l'angiogenèse, puisse être utilisée tel que définie ci-dessus.

La présente invention repose également sur la constatation faite par les Inventeurs concernant une protéine caractérisée en ce qu'elle comprend ou est constituée par :
* un fragment de la protéine NOV, représentée par la séquence SEQ ID NO : 2, sous réserve que ce fragment présente une activité d'inhibition de l'angiogenèse, ledit fragment comprenant notamment d'environ 40 à environ 180 acides aminés, et étant notamment représenté par l'une des séquences suivantes SEQ ID NO: 4, SEQ ID NO : 6, SEQ ID NO : 8, SEQ ID NO : 10 ou SEQ ID NO : 12, ou
* toute séquence dérivée de la séquence SEQ ID NO : 2 ou d'un fragment défini ci-dessus, notamment par substitution, suppression ou addition d'un ou plusieurs acides aminés, sous réserve que cette séquence dérivée présente une activité d'inhibition de l'angiogenèse, ou
* toute séquence homologue de la séquence SEQ ID NO: 2 ou d'un fragment défini ci-dessus, ayant de préférence une homologie d'au moins environ 80%, et notamment 85%, avec la région comprise entre les acides aminés en position (33) et (338) de la séquence SEQ ID NO : 2, sous réserve que cette séquence homologue présente une activité d'inhibition de l'angiogenèse,
pour la préparation d'un médicament destiné au traitement de pathologies nécessitant l'inhibition de la prolifération endothéliale, notamment dans le cadre des pathologies suivantes : la dégénérescence maculaire liée à l'âge, les rétinopathies diabétiques, la polyarthrite rhumatoïde, les angiomes, les angiosarcomes, en particulier la maladie de Castelman et le sarcome de Kaposi, puisse être utilisée tel que définie ci-dessus.

La présente invention repose également sur la constatation faite par les Inventeurs concernant une protéine caractérisée en ce qu'elle comprend ou est constituée par :
* un fragment de la protéine NOV, représentée par la séquence SEQ ID NO: 2, sous réserve que ce fragment présente une activité d'inhibition de l'angiogenèse, ledit fragment comprenant notamment d'environ 40 à environ 180 acides aminés, et étant notamment représenté par l'une des séquences suivantes SEQ ID NO : 4, SEQ NO : 6, SEQ ID NO : 8, SEQ ID NO : 10 ou SEQ ID NO : 12, ou
* toute séquence dérivée de la séquence SEQ ID NO : 2 ou d'un fragment défini ci-dessus, notamment par substitution, suppression ou addition d'un ou plusieurs acides aminés, sous réserve que cette séquence dérivée présente une activité d'inhibition de l'angiogenèse, ou
* toute séquence homologue, de la séquence SEQ ID NO : 2 ou d'un fragment défini ci-dessus, ayant de préférence une homologie d'au moins environ 80%, et notamment 85%, avec la région comprise entre les acides aminés en position (33) et (338) de la séquence SEQ ID NO : 2, sous réserve que cette séquence homologue présente une activité d'inhibition de l'angiogenèse, puisse être utilisée
pour la préparation d'un médicament destiné au traitement de pathologies nécessitant l'inhibition de l'activation endothéliale, notamment dans le cadre des pathologies suivantes : le rejet d'allogreffe et de xénogreffe, les acrocyanoses, les sclérodermies, ou dans le cadre de la préparation de greffons entre le prélèvement et la transplantation.

La présente invention repose également sur l'observation faite par les Inventeurs qu'une protéine caractérisée en ce qu'elle comprend ou est constituée par :
* un fragment de la protéine NOV, représentée par la séquence SEQ ID NO : 2, sous réserve que ce fragment présente une activité d'inhibition de l'angiogenèse, ledit fragment comprenant notamment d'environ 40 à environ 180 acides aminés, et étant notamment représenté par l'une des séquences suivantes SEQ ID NO: 4, SEQ ID NO : 6, SEQ ID NO : 8, SEQ ID NO : 10 ou SEQ ID NO : 12, ou
* toute séquence dérivée de la séquence SEQ ID NO : 2 ou d'un fragment défini ci-dessus, notamment par substitution, suppression ou addition d'un ou plusieurs acides aminés, sous réserve que cette séquence dérivée présente une activité d'inhibition de l'angiogenèse, ou
* toute séquence homologue de la séquence SEQ ID NO : 2 ou d'un fragment défini ci-dessus, ayant de préférence une homologie d'au moins environ 80%, et notamment 85%, avec la région comprise entre les acides aminés en position (33) et (338) de la séquence SEQ ID NO : 2, sous réserve que cette séquence homologue présente une activité d'inhibition de l'angiogenèse, puisse être utilisée par
pour la préparation d'un médicament destiné au traitement des cancers.

Aussi, l'invention concerne l'utilisation d'une protéine caractérisée en ce qu'elle est constituée par un fragment de la protéine NOV, ledit fragment étant représenté par la séquence SEQ ID NO : 12, pour la préparation d'un médicament destiné au traitement des cancers

Egalement l'utilisation :
- d'une protéine caractérisée en ce qu'elle comprend ou est constituée par :
   * la protéine NOV, représentée par la séquence SEQ ID NO : 2, ou
   * un fragment de cette protéine, sous réserve que ce fragment présente une activité d'inhibition de l'angiogenèse, ledit fragment comprenant notamment d'environ 40 à environ 80 acides aminés, et étant notamment représenté par l'une des séquences suivantes SEQ ID NO : 4, SEQ ID NO : 6, SEQ ID NO : 8 ou SEQ ID NO : 10, ou
   * toute séquence dérivée de la séquence SEQ ID NO : 2 ou d'un fragment défini ci-dessous, notamment par substitution, suppression ou addition d'un ou plusieurs acides aminés, sous réserve que cette séquence dérivée présente une activité d'inhibition de l'angiogenèse, ou
   * toute séquence homologue de la séquence SEQ ID NO :2 ou d'un fragment défini ci-dessous, ayant de préférence une homologie d'au moins environ 80%, et notamment 85%, avec la région comprise entre les acides aminés en position (33) et (338) de la séquence SEQ ID NO : 2, sous réserve que cette séquence homologue présente une activité d'inhibition de l'angiogenèse,
- d'une séquence nucléotidique caractérisée en ce qu'elle, comprend ou est constituée par une séquence nucléotidique codant :
   * soit pour la protéine NOV telle que définie ci-dessus,
   * soit pour un fragment de la protéine NOV tel que défini ci-dessus,
   * soit pour une séquence dérivée de la protéine NOV telle que définie ci-dessus,
   * soit pour une séquence homologue de la protéine NOV telle que définie ci-dessus,
      ladite séquence nucléotidique, correspondant notamment à la séquence nucléotidique SEQ ID NO : 1 codant pour SEQ ID NO : 2, ou à la séquence SEQ ID NO : 3 codant pour SEQ ID NO : 4, ou à la séquence SEQ ID NO : 5 codant pour SEQ ID NO : 6, ou à la séquence SEQ ID NO : 7 codant pour SEQ ID NO : 8, ou à la séquence SEQ ID NO : 9 codant pour SEQ ID NO: 10,
- d'un anticorps anti-idiotypique de la protéine NOV, peut être utilisée pour la préparation d'un médicament destiné au traitement de pathologies nécessitant l'inhibition de la prolifération endothéliale, notamment dans le cadre des pathologies suivantes : les cancers, la dégénérescence maculaire liée à l'âge, les rétinopathies diabétiques, la polyarthrite rhumatoïde, les angiomes, les angiosarcomes, en particulier la maladie de Castelman et le sarcome de Kaposi.

De la même manière,
- une protéine caractérisée en ce qu'elle comprend ou est constituée par :
   * la protéine NOV, représentée par la séquence SEQ ID NO : 2, ou
   * un fragment de cette protéine, sous réserve que ce fragment présente une activité d'inhibition de l'angiogenèse, ledit fragment comprenant notamment d'environ 40 à environ 80 acides aminés, et étant notamment représenté par l'une des séquences suivantes SEQ ID NO: 4, SEQ ID NO : 6, SEQ ID NO : 8 ou SEQ ID NO : 10, ou
   * toute séquence dérivée de la séquence SEQ ID NO : 2 ou d'un fragment défini ci-dessous, notamment par substitution, suppression ou addition d'un ou plusieurs acides aminés, sous réserve que cette séquence dérivée présente une activité d'inhibition de l'angiogenèse, ou
   * toute séquence homologue de la séquence SEQ ID NO : 2 ou d'un fragment défini ci-dessous, ayant de préférence une homologie d'au moins environ 80%, et notamment 85%, avec la région comprise entre les acides aminés en position (33) et (338) de la séquence SEQ ID NO: 2, sous réserve que cette séquence homologue présente une activité d'inhibition de l' angiogenèse,
- une séquence nucléotidique caractérisée en ce qu'elle comprend ou est constituée par une séquence nucléotidique codant :
   * soit pour la protéine NOV telle que définie ci-dessus,
   * soit pour un fragment de la protéine NOV tel que défini ci-dessus,
   * soit pour une séquence dérivée de la protéine NOV telle que définie ci-dessus
   * soit pour une séquence homologue de la protéine NOV telle que définie ci-dessus, 1
      ladite séquence nucléotidique correspondant notamment à la séquence nucléotidique SEQ ID NO : 1 codant pour SEQ ID NO : 2, ou à la séquence SEQ ID NO : 3 codant pour SEQ ID NO : 4, ou à la séquence SEQ ID NO : 5 codant pour SEQ ID NO : 6, ou à la séquence SEQ ID NO : 7 codant pour SEQ ID NO : 8, ou à la séquence SEQ ID NO : 9 codant pour SEQ ID NO : 10,
- un anticorps anti-idiotypique de la protéine NOV,
peuvent être utilisés pour la préparation d'un médicament destiné au traitement de pathologies nécessitant l'inhibition de l'activation endothéliale, notamment dans le cadre des pathologies suivantes : le rejet d'allogréffe et de xénogreffe, les acrocyanoses, les sclérodermies, ou dans le cadre de la préparation de greffons entre le prélèvement et la transplantation.

Les Inventeurs ont également constaté qu'il était possible de préparer une composition pharmaceutique caractérisée en ce qu'elle contient à titre de substance active :
- une protéine caractérisée en ce qu'elle comprend ou est constituée par :
   * la protéine NOV, représentée par la séquence SEQ ID NO : 2, ou
   * un fragment de cette protéine, sous réserve que ce fragment présente une activité d'inhibition de l'angiogenèse, ledit fragment comprenant notamment d'environ 40 à environ 180 acides aminés, de préférence d'environ 40 à environ 80 acides aminés, et étant notamment représenté par l'une des séquences suivantes SEQ ID NO : 4, SEQ ID NO : 6, SEQ ID NO : 8, SEQ ID NO : 10 ou SEQ ID NO : 12, ou
   * toute séquence dérivée de la séquence SEQ ID NO : 2 ou d'un fragment défini ci-dessous, notamment par substitution, suppression ou addition d'un ou plusieurs acides aminés, sous réserve que cette séquence dérivée présente une activité d'inhibition de l'angiogenèse, ou
   * toute séquence homologue de la séquence SEQ ID NO : 2 ou d'un fragment défini ci-dessous, ayant de préférence une homologie d'au moins environ 80%, et notamment 85%, avec la région comprise entre les acides aminés en position (33) et (338) de la séquence SEQ ID NO : 2, sous réserve que cette séquence homologue présente une activité d'inhibition de l'angiogenèse,
- une séquence nucléotidique caractérisée en ce qu'elle comprend ou est constituée par une séquence nucléotidique codant ;
   * soit pour la protéine NOV telle que définie ci-dessus,
   * soit pour un fragment de la protéine NOV tel que défini ci-dessus,
   * soit pour une séquence dérivée de la protéine NOV telle que définie ci-dessus,
   * soit pour une séquence homologue de la protéine NOV telle que définie ci-dessus,
   ladite séquence nucléotidique correspondant notamment à la séquence nucléotidique SEQ ID NO : 1 codant pour SEQ ID NO : 2, ou à la séquence SEQ ID NO : 3 codant pour SEQ ID NO : 4, ou à la séquence SEQ ID NO : 5 codant pour SEQ ID NO : 6, ou à la séquence SEQ ID NO: 7 codant pour SEQ ID NO : 8, ou à la séquence SEQ ID NO : 9 codant pour SEQ ID NO : 10, ou à la séquence SEQ ID NO : 11 codant pour SEQ ID NO : 12,
- un anticorps anti-idiotypique de là protéine NOV,
en association avec un vecteur pharmaceutiquement acceptable.

Egalement, il est possible de préparer une composition pharmaceutique caractérisée en ce qu'elle contient à titre de substance active une protéine caractérisée en ce qu'elle comprend ou est constituée par :
* un fragment de la protéine NOV, représentée par la séquence SEQ ID NO : 2, sous réserve que ce fragment présente une activité d'inhibition de l'angiogenèse, ledit fragment comprenant notamment d'environ 40 à environ 180 acides aminés, et étant notamment représenté par l'une des séquences suivantes SEQ ID NO: 4, SEQ ID NO *-* 6, SEQ ID NO : 8, SEQ ID NO : 10 ou SEQ ID NO : 12, ou
* toute séquence dérivée de la séquence SEQ ID NO : 2 ou d'un fragment défini ci-dessus, notamment par substitution, suppression ou addition d'un ou plusieurs acides aminés, sous réserve que cette séquence dérivée présente une activité d'inhibition de l'angiogenèse, ou
* toute séquence homologue, de la séquence SEQ ID NO : 2 ou d'un fragment défini ci-dessus, ayant de préférence une homologie d'au moins environ 80%, et notamment 85%, avec la région comprise entre les acides aminés en position (33) et (138) de la séquence SEQ ID NO: 2, sous réserve que cette séquence homologue présente une activité d'inhibition de l'angiogenèse,
en association avec un vecteur pharmaceutiquement acceptable.

L'invention concerne ainsi une vomposition pharmaceutique caractérisée en ce qu'elle contient à titre de substance active :
- une protéine caractérisée en ce qu'elle est constituée par un fragment de la protéine NOV, représentée par la séquence SEQ ID NO:2, ledit fragment étant représenté par la séquence SEQ ID NO : 12
- une séquence nucléotidique caractérisée en ce qu'elle comprend ou est constituée par une séquence nucléotidique codant :
   * soit pour la protéine NOV telle que définie ci-dessus,
   * soit pour un fragment de la protéine NOV tel que défini ci-dessus,
   ladite séquence nucléotidique correspondant à la séquence nucléotidique SEQ ID NO : 1 codant pour SEQ ID NO : 2, ou à la séquence SEQ ID NO : 11 codant pour SEQ ID NO : 12
- un anticorps anti-idiotypique de la protéine NOV représentée par la séquence SEQ ID NO : 2, ledit anticorps mimant les fonctions de la protéine NOV en reconnaissant le VEGF,
en association avec un vecteur pharmaceutiquement acceptable.

Dans un mode de réalisation avantageux, l'invention concerne une composition pharmaceutique définie ci-dessus, caractérisée en ce qu'elle contient à titre de substance active une protéine caractérisée en ce qu'elle est constituée par un fragment de la protéine NOV, représéntée par la séquence SEQ ID NO : 2, ledit fragment étant représenté par la séquence SEQ ID NO : 12, en association avec un vecteur pharmaceutiquement acceptable.

Egalement, il peut être préparé une composition pharmaceutique avantageuse qui contient, à titre de substance active, le fragment TSP-1 susmentionné (SEQ ID NO : 8).

Une composition avantageuse selon l'invention est caractérisée en ce que l'activité d'inhibition de l'angiogenèse est mesurée selon le test de prolifération, de migration ou de différenciation, et en ce que cette activité d'inhibition correspond à un pourcentage d'inhibition compris de 20% à 100% de l'angiogenèse obtenue en présence du véhicule seul.

Les tests de prolifération, de migration et de différenciation (angiogenèse *in vitro*)+ sont décrits plus loin dans la partie expérimentale.

La présente invention concerne également une composition telle que définie ci-dessus, caractérisée en ce qu'elle contient à titre de substance active la protéine NOV, représentée par la séquence SEQ ID NO : 2.

Selon un mode de réalisation avantageux de la présente invention, la composition telle que définie ci-dessus est caractérisée en ce qu'elle est susceptible d'être administrée à raison d'environ 0,1 à environ 20 mg/kg/jour.

La présente invention concerne également l'utilisation telle que définie ci-dessus, pour la préparation d'une composition telle que définie ci-dessus, destinée à être administrée à raison d'environ 0,1 à environ 20 mg/kg/jour.

Il est aussi possible de préparer une composition telle que définie ci-dessus caractérisée en ce qu'elle est administrée sous forme d'un gène, d'une protéine ou d'un peptide contenant la séquence de type TSP-1 (SEQ ID NO : 8).

Une composition avantageuse de l'invention est notamment administrée de préférence sous forme injectable.

### DESCRIPTION DES FIGURES

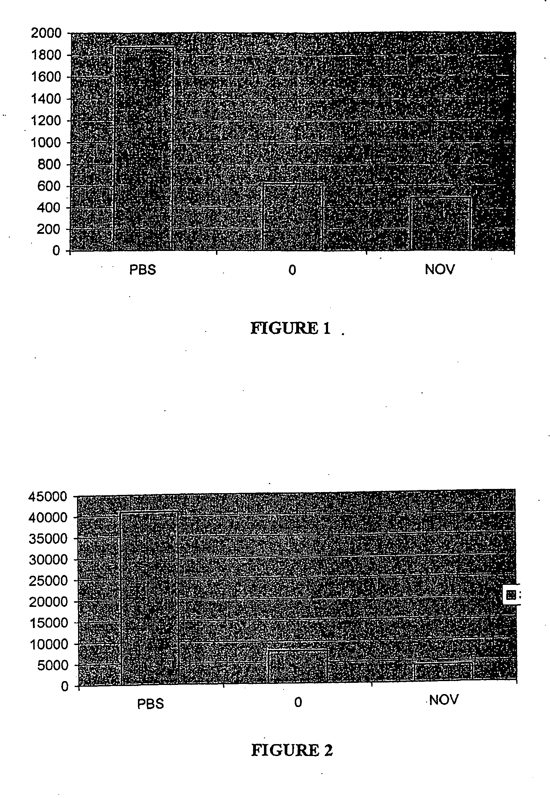

La Figure 1 correspond à la liaison de la forme iodée du VEGF₁₆₅ sur la protéine NOV.

La protéine NOV (4 µg/ml) est immobilisée sur du plastique selon les conditions décrites dans la partie expérimentale, puis incubée avec du VEGF₁₆₅ iodé (1 ng/puits) en absence (colonne PBS) ou présence de 2 µg/ml de VEGF₁₆₅ (colonne 0) ou de NOV (colonne NOV). Les résultats sont exprimés en cpm de VEGF₁₆₅ iodé fixé par puits, après lavage.

La Figure 2 correspond à la liaison de la forme iodée du VEOF₁₈₉ sur la protéine NOV.

La protéine NOV (4 µg/ml) est immobilisée sur du plastique selon les conditions décrites dans la partie expérimentale, puis incubée avec du VEGF₁₈₉ iodé (1 ng/puits) en absence (colonne PBS) ou présence de 2 µg/ml de VEGF₁₈₉, (colonne 0) ou de NOV (colonne NOV). Les résultats sont exprimés en cpm de VEGF₁₈₉ iodé fixé par puits, après lavage.

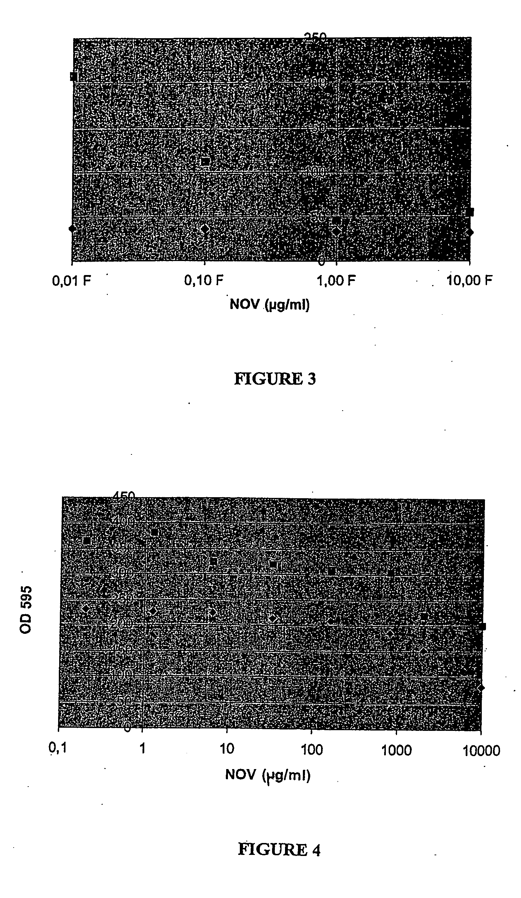

La Figure 3 correspond au test de migration des cellules. Les cellules sont comptées dans 8 champs et la moyenne est représentée sur l'axe des ordonnées. L'axe des abscisses correspond à la concentration de la protéine NOV en µg/ml. Les points représentés par des losanges correspondent aux cellules non incubées avec VEGF et les points représentés par des carrés correspondent aux cellules préalablement traitées avec du VEGF.

La Figure 4 correspond au test de prolifération des cellules. L'axe des abscisses correspond à la concentration de la protéine NOV en µg/ml et l'axe des ordonnées à la densité optique mesurée à 595 nm. Les points représentés par des losanges correspondent aux cellules qui n'ont pas été stimulées par le VEGF et les points représentés par des carrés correspondent aux cellules qui ont été stimulées par le VEGF.

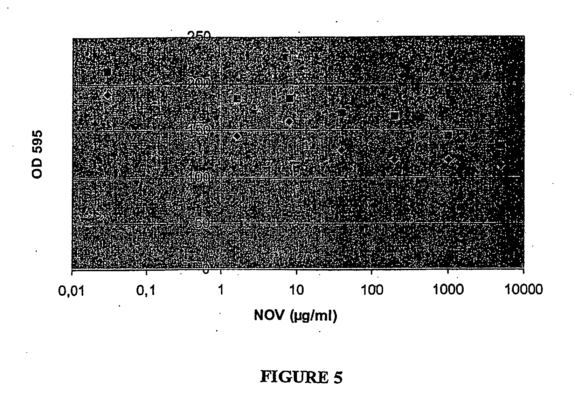

La Figure 5 correspond au test d'adhésion des cellules FBAE. L'axe des abscisses correspond à la concentration de la protéine NOV en µg/ml et l'axe des ordonnées à la densité optique mesurée à 595 nm. Les points représentés par des losanges correspondent aux cellules non incubées avec VEGF et les points représentés par des carrés correspondent aux cellules préalablement traitées avec du VEGF.

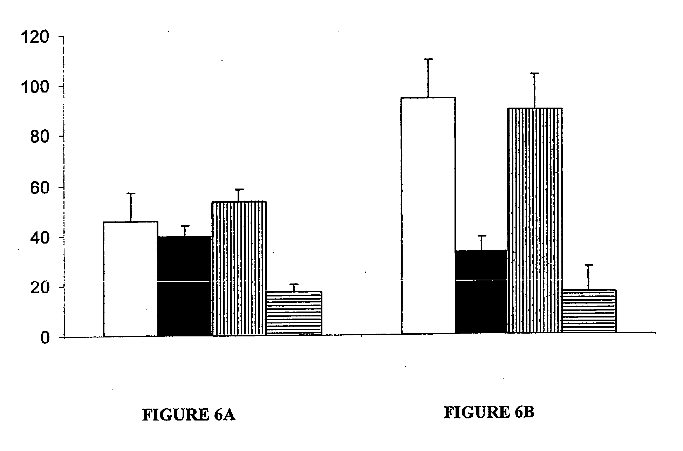

Les Figures 6A et 6B représentent l'effet de NOV et de ses fragments sur la migration des cellules HUAEC stimulées avec VEGF₁₆₅. La Figure 6A correspond aux essais témoins avec des cellules non stimulées avec VEGF₁₆₅ et la Figure 6B correspond aux essais avec des cellules stimulées avec VEGF₁₆₅. Les colonnes représentent le nombre de cellules/champs. Les colonnes blanches correspondent aux cellules témoins (sans ajout de NOV ou de l'un de ses fragments) ; les colonnes noires correspondent aux cellules stimulées en présence de NOV; les colonnes hachurées verticalement correspondent aux cellules stimulées en présence du fragment N-terminal de NOV (acides animés 1 à 187 de NOV) et les colonnes hachurées horizontalement correspondent aux cellules stimulées en présence du fragment C-terminal de NOV.

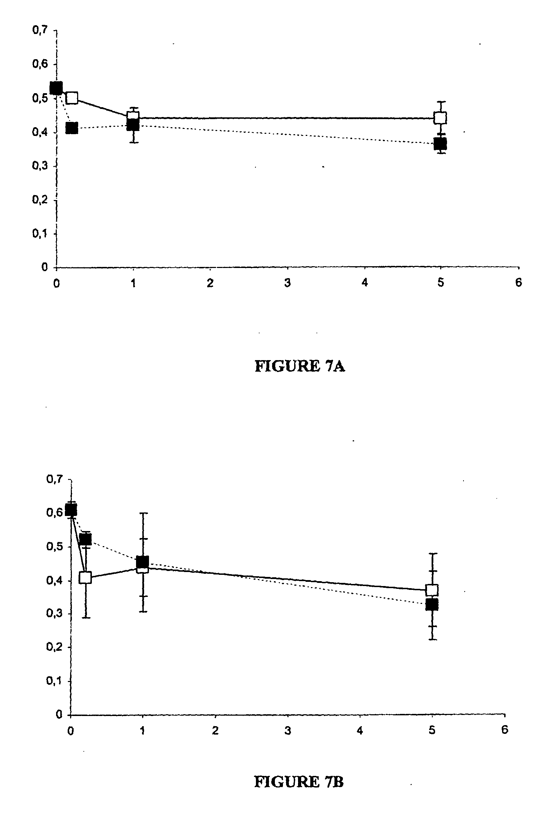

La Figure 7A représente l'effet de la protéine NOV ou de son fragment C-terminal sur la prolifération des HUAEC (cellules endothéliales artérielles ombilicales humaines) stimulées avec VEGF₁₆₅. L'axe des abscisses correspond à la concentration de la protéine NOV ou du fragment C-terminal (SEQ ID NO 12) en µg/ml et l'axe des ordonnées à la densité optique mesurée à 595 nm. La courbe en trait plein avec les carrés blancs correspond à la protéine NOV et la courbe en trait pointillé avec les carrés noirs correspond au fragment SEQ ID NO : 12.

La Figure 7B représente l'effet de la protéine NOV ou de son fragment C-terminal sur la prolifération des HUAEC stimulées avec bFGF. L'axe des abscisses correspond à la concentration de la protéine NOV ou du fragment C-terminal (SEQ ID NO : 12) en µg/ml et l'axe des ordonnées à la densité optique mesurée à 595 nm. La courbe en trait plein avec les carrés blancs correspond à la protéine NOV et la courbe en trait pointillé avec les carrés noirs correspond au fragment SEQ ID NO : 12.

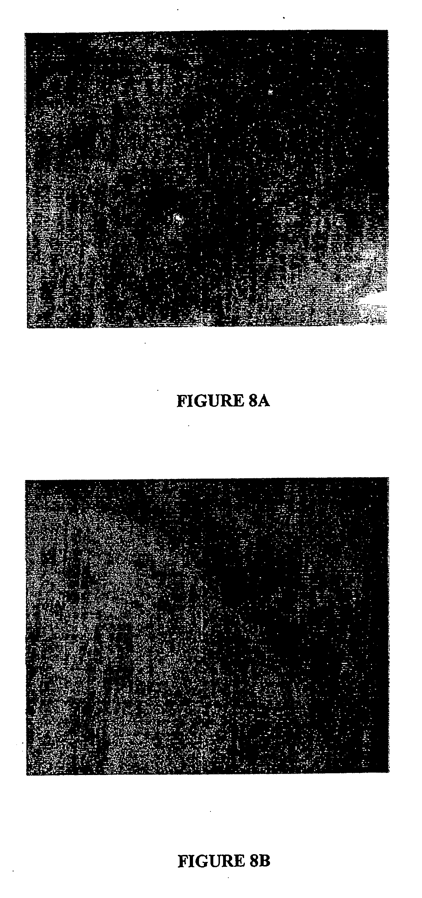

Les Figures 8A et 8B représentent l'effet du fragment C-terminal de la protéine NOV (SEQ ID NO : 12) sur l'angiogenèse cornéenne. La Figure 8A correspond à l'injection de LPS seul et la Figure 8B à l'injection de LPS et dudit fragment C-terminal.

### PARTIE EXPÉRIMENTALE

### Matériels :

La molécule NOV est produite par infection de cellules d'insecte SF9 par un baculovirus recombinant contenant l'ADNc correspondant (SEQ ID NO : 1)(Thibout et al., 2003).

Les isoformes de 165 et 189 acides aminés du VEGF sont produites par infection de cellules d'insecte SF9 par un baculovirus recombinant contenant l'ADNc correspondant (Plouët et al., 1997).

Des cellules endothéliales artérielles ombilicales humaines (HUAEC) ont été isolées à partir d'artères ombilicales perfusées avec du collagène (Sigma) pour digérer la membrane basale, Les cellules HUAEC ont été maintenues dans du SFM (Life Sciences) additionné de 20% de sérum de veau foetal (SVF) inactivé par la chaleur. Les cultures souches ont reçu 1 ng/ml de VEGF chaque jour.

Des cellules endothéliales d'aorte foetale bovine (FBAE) ont été isolées à partir d'aortes foetales obtenues auprès d'un abattoir local. Les cellules ont été maintenues dans du DMEM glutamax (Life Sciences) additionné de 10% de sérum de veau nouveau-né (NBCS) inactivé par la chaleur, 100 µg/ml de pénicilline et 100 µg/ml de streptomycine à 37°C dans 10% de CO₂ et ng/ml de VEGF tous les 2 jours.

### Interaction directe entre VEGF et NOV

Pour la liaison à la protéine NOV immobilisée, des plaques ELISA à 96 puits ont été recouvertes de 4 µg/ml de protéine NOV dans un tampon carbonate 0,05 M à pH 9,6 pendant la nuit à 4°C. Les sites de liaison non spécifiques ont été bloqués avec 5 mg/ml de BSA dans du tampon carbonate. Après avoir lavé deux fois les puits avec du PBS à pH 7,4, on a ajouté 1 ng de VEGF iodé à chaque puits en présence ou non de 2 µg/ml de VEGF₁₆₅ ou de NOV, dilués dans du PBS contenant 0,05 % de Tween 20, 0,5% de BSA, 1 mM de MgCl₂ et 1 mM de CaCl₂.

Les puits ont été lavés 3 fois avec un mélange PBS-Tween 20 0,1%-BSA 0,5% et les protéines liées ont été solubilisées dans du NaOH 0,2M.

Les résultats de ces expériences sont représentés dans les figures 1 et 2.

La Figure 1 montre que le VEGF₁₆₅ iodé s'associe spécifiquement à NOV puisque l'addition de VEGF non radiomarqué (VEGF) inhibe cette liaison. De même, l'addition de NOV inhibe la liaison de VEGF₁₆₅ radiomarqué à NOV.

### Tests de migration

Des cellules FBAE sont inoculées dans des puits de 4 cm² à haute densité (50 000 cellules/puits). Quand la monocouche est confluente, la prolifération est arrêtée par l'incubation, pendant une nuit, en présente de DMEM sans sérum. Une blessure est alors pratiquée dans la monocouche à l'aide d'un grattoir mousse, permettant de délimiter une surface libre de toute cellule. Les monocouches sont ensuite lavées 3 fois par du DMEM pour enlever les cellules non adhérentes. Une photographie est alors prise pour délimiter la surface avant toute migration cellulaire. Les puits sont ensuite incubés en DMEM seul ou en présence de 50 ng/ml de VEGF en présence de concentrations variables de NOV. Après 24 h les puits sont lavés 3 fois et colorés au May-Grunwald-Giemsa et photographiées. Les photographies prises avant et après l'expérience sont alors superposées pour permettre le comptage des cellules ayant migré.

Les résultats de ces tests sont indiqués dans la Figure 3.

L'addition de NOV en l'absence de VEGF n'a pas d'effet sur la migration basale des cellules. En revanche, NOV inhibe l'activité du VEGF et 50% de l'effet maximal est obtenu avec une concentration de 50-100 ng/ml de NOV.

### Tests de prolifération

Des plaques de culture à 96 puits ont été ensemencées avec 1000 cellules FBAE par puits dans du DMEM additionné de 5% de NBCS. Les cellules ont été stimulées ou non avec 2 ng/ml de VEGF₁₆₅ et différentes concentrations de NOV. Au bout de 5 jours, les puits ont été rincés doucement avec du DMEM et les cellules ont été fixées dans 1% de glutaldéhyde pendant 20 minutes à température ambiante. Les cellules fixées ont été quantifiées par incorporation de violet cristallisé (Kueng et al., 1989) : les cellules ont été incubées dans 0,1% de violet cristallisé (Sigma) dilué dans 0,2 M de tampon borate à pH 9,5 pendant 20 minutes à température ambiante, le colorant non incorporé a été éliminé en lavant complètement les puits avec de grandes quantités d'eau et le colorant violet cristallisé incorporé a ensuite été solubilisé par 100 µl de 10% d'acide acétique par puits. Les lectures de densité optique ont été effectuées à 595 nm. Des résultats similaires ont été obtenus dans trois expériences séparées (voir Figure 4). Les valeurs indiquées sont des densités optiques moyennes de 6 puits ± SD.

La protéine NOV utilisée seule n'a pas d'effet significatif sur la prolifération basale (due au sérum seul). En revanche, la protéine NOV inhibe la prolifération induite par le VEGF sur un mode dépendant de la dose. 50% de l'effet maximal est obtenu avec une concentration de 100-200 ng/ml de NOV.

### Tests d'adhésion cellulaire

Des plaques ELISA à 96 puits (Nunc) ont été recouvertes de protéine VEGF₁₆₅ selon le protocole décrit dans l'article de Hutchings et al. (2003), diluée dans du tampon carbonate 0,05 M à pH 9,6 pendant la nuit à 4°C. Les sites de liaison non spécifiques ont été bloqués pendant 1 heure à 37°C avec 5 mg/ml de BSA dans du tampon carbonate et lavés deux fois avec du DMEM avant lés expériences. Les cellules ont été trypsinisées, lavées et remises en suspension dans 5 ml de DMEM avec 10% de SVF dans un tube de plastique non traité et incubées pendant 1 heure à 37°C avec 10% de CO₂. Les cellules ont ensuite été concentrées par centrifugation et remises en suspension dans un mélange DMEM + 0,2% BSA sans sérum et la suspension cellulaire a été traitée pendant 20 minutes (37°C, 10% de CO₂) avec la protéine NOV utilisée pour moduler l'adhésion. 40 000 cellules par puits ont été distribuées dans les puits dans un volume de 100 µl de DMEM + 0,2% de BSA. Les cellules ont été laissées adhérer à 37°C sous 10% de CO₂ pendant le temps voulu. Les puits ont été lavés doucement trois fois avec du DMEM pour retirer les cellules non adhérentes et les cellules adhérentes ont été fixées avec 1% de glutaraldéhyde pendant 20 minutes à température ambiante. Les cellules fixées ont été quantifiées par incorporation de cristal violet (Kueng et al., 1989) : les cellules ont été incubées avec 0,1% de violet cristallisé (Sigma) dilué dans 0,2 M de tampon borate à pH 9,5 pendant 20 minutes à température ambiante, le colorant non incorporé a été éliminé en lavant complètement les puits avec de grandes quantités d'eau et le colorant cristal violet incorporé a ensuite été solubilisé par 100 µl de 10% d'acide acétique par puits (voir Figure 5).

### Angiogenése in vitro

Quatre queues de rat ont été dépiautées et disséquées pour récupérer les faisceaux blancs qui sont constitués en majeure partie de collagène de type I. Le collagène est extrait de ces fibres dans 50 ml d'acide acétique 0,5 M froid et agités sur une nuit Le liquide est ensuite centrifugé à 5000g pendant 40 minutes et le surnageant est récupéré. L'extraction est refaite une fois avec 20 ml d'acide acétique, les surnageants sont mélangés et puis dialysés contre 1 1 d'acide acétique 0,2 M. La concentration en collagène est ajustée à 3 mg/ml par pesée. La préparation des gels pour l'angiogenèse *in vitro* s'effectue sur de la glace pour conserver la solution de collagène sous forme liquide. Un ml de collagène (5 mg/ml) est mélangé à 0,5 ml de DMEM 10X (contenant une concentration 10X en antibiotiques et en glutamine), 0,9 ml de H₂O stérile et 0,1 ml de bicarbonate de sodium 1M. Une fois le pH ajusté à 7,4, il est ajouté un volume égal de matrigel (Becton Dickinson). Le gel est coulé dans des puits de culture (2 mm d'épaisseur) et incubé à 37°C pour se solidifier. Les cellules sont rajoutées après 15 minutes (100 000 cellules/cm²) sur la surface du gel. Après 2 heures, les différents facteurs solubles sont ajoutés et les cellules sont observées et photographiées après 24 heures.

### Production d'anticorps anti-idiotypiques

Dans un premier temps on prépare un anticorps neutralisant de NOV en injectant à un animal, notamment une souris, de la protéine NOV mélangée avec de l'adjuvant complet de Freund (1 volume par volume de protéine NOV). On choisira une quantité de NOV comprise entre 1 et 200 µg/kg de poids corporel pour immuniser l'animal. La même opération est effectuée à 15 et 30 jours d'intervalle, excepté que l'adjuvant complet est remplacé par de l'adjuvant incomplet. Au jour 40 une saignée est pratiquée, le sérum est séparé et les immunoglobulines sont purifiées par toute méthode de fractionnement habituelle, notamment précipitation au sulfate d'ammonium, chromatographie d'affinité pour la protéine A ou G. On mesure l'activité neutralisante des immunoglobulines par n'importe quel test décrit (liaison du VEGF iodé, prolifération, migration, adhésion cellulaire). Un lot d'immunoglobulines sera dit neutralisant quand il aura la capacité d'inhiber l'interaction de NOV avec le VEGF.

Dans un deuxième temps on prépare des anticorps anti-idiotypiques de NOV en injectant à des souris par voie sous-cutanée 1-100 µg de la préparation des immunoglobulines neutralisant l'activité de NOV précédemment décrites en association avec 100 µl d'adjuvant, notamment de l'adjuvant complet de Freund (Sigma). L'injection est répétée 15, 30 et 45 jours après. Cinquante-cinq jours après la première injection, on injecte à des souris 10 µg du même anticorps par voie intrapéritonéale. Cinquante-huit jours après la première injection, les souris sont sacrifiées et leurs rates sont prélevées et dilacérées dans du milieu ISCOVE pour libérer les splénocytes. Les splénocytes sont fusionnés avec des cellules de myélome de souris, notamment des cellules AG8X 63 (Keamey et al., 1979), et incubés à raison de 100 000 cellules/puits. La fusion s'effectue par ajout de 20 fois 50 µl de polyéthylène glycol (PEG) à 30 secondes d'intervalle. Quatre ml de milieu ISCOVE préchauffé à 37°C sont alors ajoutés goutte à goutte sur la suspension cellulaire, puis après une période d'incubation de 4 minutes à 37°C, 4 ml sont ajoutés. La suspension est centrifugée puis le culot cellulaire est alors repris dans 100 ml de milieu ISCOVE complémenté avec 20% de sérum de veau foetal et du HAT 1X (50X : Hypoxanthine 5 mM, Aminoptérine 20 µM et Thymidine 0,8 mM) et distribuées à raison de 100 µl par puits sur les macrophages. Après 5 jours, 100 µl de milieu HAT sont ajoutés, et entre 8 et 14 jours le milieu conditionné de chaque hybridome est prélevé pour mesurer par ELISA les anticorps dirigés contre les anticorps ayant servi d'agent immunogène, c'est à dire les anticorps anti-NOV. On mesure alors l'activité des anticorps anti-idiotypiques par un test ELISA :

Les fragments Fab des immunoglobulines anti NOV, préparés par toute technique conventionnelle, notamment une digestion à la papaïne, sont immobilisés sur des plaques de microtitration (0,1-20 µg/ml dans du tampon carbonate 50 mM pH 9,6). Après saturation des sites non spécifiques par une solution de sérum albumine diluée à 5 mg/ml dans le même tampon, les surnageants de cultures d'hybridomes sont ajoutés dilués pour moitié dans du tampon PBS contenant 0,05% de Tween 20. Après rinçages, les anticorps anti-idiotypiques sont révélés par adjonction d'une concentration appropriée d'anticorps anti-Fc de souris couplé à la peroxydase. La quantité d'anticorps anti-idiotypique fixé est alors mesurée par révélation de la peroxydase et est proportionnelle à l'intensité de la réaction colorimétrique.

Les hybridomes sélectionnés par leur capacité à sécréter des anticorps dirigés contre des anticorps anti-NOV sont alors clonés, c'est-à-dire que les cellules sont ensemencées en condition de dilution limite (5 cellules/ml) sous un volume de 0,1 ml par puits. Le milieu est changé après 10 jours. Après 15 jours, certains puits contiennent des foyers de cellules qui se sont multipliées à partir de la cellule ensemencée au départ, donc toutes ces cellules sont identiques et sont issues du même clone. Quand la surface occupée par les cellules représente au moins la moitié de la surface totale du puits, le milieu est prélevé et analysé comme précédemment par un ELISA sur Fab anti-NOV. A ce stade on peut sélectionner les clones producteurs d'anticorps et connaître leur spécificité.

Une fois les clones identifiés, leur nature monoclonale est affirmée par l'opération classique consistant à ensemencer une plaque de 96 puits avec des cellules issues du même clone diluées en conditions limites comme précédemment. Les clones sécréteurs doivent donc tous sécréter un anticorps de même spécificité pour que l'on déclare cet anticorps monoclonal. Un troisième clonage est alors effectué exactement dans les mêmes conditions pour s'assurer que les clones sont bien monoclonaux.

Les anticorps anti-idiotypiques sont criblés par une batterie de tests, notamment par un test ELISA sur VEGF immobilisé. Du VEGF est immobilisé (0,1-10 µg/ml) dans du tampon carbonate comme précédemment et toutes les étapes de cet ELISA sont identiques à celles décrites dans l'ELISA sur Fab anti-NOV. Ce test permet de cribler parmi tous les anticorps anti-idiotypiques ceux qui miment les fonctions de la protéine NOV (SEQ ID NO : 2) ou des fragments le type TSP-1 (SEQ ID NO : 8), c'est-à-dire des anticorps reconnaissant le VEGF.

### CONSTRUCTION DE MUTANTS DE NOV

Des mutants de délétion de la protéine NOV ont été construits selon la référence (Perbal et al., 1999) et produits dans un système d'expression de baculovirus :
- N-Ter (correspond à une séquence comprenant les acides aminés 1-187 de NOV) et
- C-Ter contenant les acides aminés 188 à 357 (cette séquence contient le domaine de type thrombospondine (SEQ ID NO : 8) et le domaine C-terminal riche en cystéines (SEQ ID NO : 10).

### Tests de migration (Figure 6)

Des cellules HUAEC sont inoculées dans des puits de 4 cm² à haute densité (50 000 cellules/puits). Quand la monocouche est confluente, la prolifération est arrêtée par l'incubation, pendant une nuit, en présence de SFM avec 1% de NBCS. Une blessure est alors pratiquée dans la monocouche à l'aide d'un grattoir mousse, permettant de délimiter une surface libre de toute cellule. Les monocouches sont ensuite lavées 3 fois par du SFM pour enlever les cellules non adhérentes. Une photographie est alors prise pour délimiter la surface avant toute migration cellulaire. Les puits sont ensuite incubés en SFM seul ou en présence de 50 ng/ml de VEGF en présence de concentrations variables de NOV ou de ses fragments N-Ter ou C-Ter. Après 24 h les puits sont lavés 3 fois et colorés au May-Grunwald-Giemsa et photographiés. Les photographies prises avant et après l'expérience sont alors superposées pour permettre le comptage des cellules ayant migré.

Les résultats de ces tests sont indiqués dans la Figure 6.

L'addition de NOV ou du fragment N-Ter en l'absence de VEGF n'a pas d'effet sur la migration basale des cellules. NOV inhibe l'activité du VEGF et 50% de l'effet maximal est obtenu avec une concentration de 50-100 ng/ml de NOV. Le fragment N-Ter n'exerce pas d'activité inhibitrice. En revanche, le fragment C-Ter inhibe la migration des cellules HUAEC.

Ces expériences démontrent que la séquence de NOV comprenant les acides aminés 188 à 357 est bien responsable de l'activité d'inhibition de l'angiogenèse due au VEGF et qu'elle induit une activité inhibant la migration y compris en absence de VEGF.

### Tests de prolifération (Figure 7)

Des plaques de culture à 96 puits ont été ensemencées avec 2000 cellules HUAEC par puits dans du milieu SFM additionné de 10% de NBCS. Les cellules ont été stimulées ou non avec 2 ng/ml de VEGF₁₆₅ et différentes concentrations de NOV ou du fragment C-Ter. Au bout de 5 jours, les puits ont été rincés doucement avec du milieu SFM et les cellules ont été fixées dans 1% de glutaldéhyde pendant 20 minutes à température ambiante. Les cellules fixées ont été quantifiées par incorporation de violet cristallisé : les cellules ont été incubées dans 0,1% de violet cristallisé (Sigma) dilué dans 0,2 M de tampon borate à pH 9,5 pendant 20 minutes à température ambiante, le colorant non incorporé a été éliminé en lavant complètement les puits avec de grandes quantités d'eau et le colorant violet cristallisé incorporé a ensuite été solubilisé par 100 µl de 10% d'acide acétique par puits. Les lectures de densité optique ont été effectuées à 595 nm. Des résultats similaires ont été obtenus dans trois expériences séparées (voir Figure 7). Les valeurs indiquées sont des densités optiques moyennes de 6 puits ± SD.

La protéine NOV inhibe la prolifération induite par le VEGF et le FGF sur un mode dépendant de la dose. 50% de l'effet maximal est obtenu avec une concentration inhibitrice de 50% de 200 ng/ml de NOV vis-à-vis du VEGF et du FGF. De même le fragment C-Ter inhibe la prolifération induite par le VEGF ou le FGF avec une concentration de 200 ng/ml.

Ces expériences démontrent que la séquence de NOV comprenant les acides aminés 188 à 357 est bien responsable de l'activité d'inhibition de l'angiogenèse. L'observation selon laquelle l'activité mitogène du FGF est aussi inhibée par le fragment NOV 188-357 démontre que l'activité inhibitrice n'est pas restreinte au seul facteur VEGF.

### Angiogenèse de cornée

Des rats Wistar sont anesthésiés. Les cornées sont incisées et une poche cornéenne est obtenue en dilacérant l'épaisseur du stroma à l'aide d'une spatule mousse. Dans le fond de la poche est inséré un implant d'Elvax (DuPont) contenant du lipopolysaccharide, un agent inflammatoire déclenchant une réaction angiogénique dépendant de plusieurs facteurs angiogéniques. Après 8 jours une réaction angiogénique est visible en regard du limbe. Quand le fragment C-Ter est injecté dans la poche cornéenne (5 µg tous les 2 jours entre J4 et J8), la réaction angiogénique est totalement inhibée (Figure 8).

Ces expériences démontrent que le fragment C-Ter de NOV exerce une activité anti-angiogénique majeure car dépendante de l'activation de plusieurs facteurs angiogéniques.

### RÉFÉRENCES

- Bork P (1993) The modular architecture of a new family of growth regulators related to connective tissue growth factor. FEBS Lett. 327: 125-130,
- Bradham DM, Igarashi A, Potter RL, Grotendorst GR (1991) Connective tissue growth factor: a cysteine-rich mitogen secreted by human vascular endothelial cells is related to the SRC-induced immediate early gene product CEF-10. J Cell Biol. 114:1285-1294,
- Brigstock DR (1999) The connective tissue growth factor/cysteine-rich 61/nephroblastoma overexpressed (CCN) family. Endocrine Rev. 20:189-206,
- Brooks PC, Clark RA, Cheresh DA (1994) Requirement of vascular integrin alpha v beta 3 for angiogenesis. Science, 264, 569-71,
- Celerier J, Cruz A, Lamande N, Gasc JM, Corvol P (2002) Angiotensinogen and its cleaved derivatives inhibit angiogenesis. Hypertension. 39(2):224-8,
- Chevalier G, Yeger H, Martinerie C, et al. (1998) nov H: Differential expression in developing kidney and in Wilms' tumors. Am J Pathol. 152:1563-1575,
- Hashimoto Y, Shindo-Okada N, Tani M, et al. (1998) Expression of the Elm-1 gene, a novel gene of the CCN (CTGF, Cyr61/Cef10 and nov) family, suppress in vivo growth and metastasis of K-1735 murine melanoma cells. J Exp Med. 187:289-296,
- Herbst et al. (2002) J. Clin. Oncol. 20:3804-3814,
- Hutchings H, Ortéga N, Plouët J (2003) Extracellular matrix bound vascular endothelial growth factor promotes endothelial cell adhesion, migration and survival through integrin ligation. FASEB J. Apr 22 (Epub ahead of print)
- Inoki I, Shiomi T, Hashimoto G, Enomoto H, Nakamura H, Makino K, Ikeda E, Takata S, Kobayashi K, Okada Y (2002) Connective tissue growth factor binds vascular endothelial growth factor (VEGF) and inhibits VEGF-induced angiogenesis. FASEB J. 16(2):219-21,
- Jain RK, Schlenger K, Hockel M, Yuan F (1997) Quantitative angiogenesis assays: progress and problems. Nat Med. 3(11):1203-8,
- Joliot V, Martinerie C, Dambrine G, et al. (1992) Proviral rearrangements and overexpression of a new cellular gene (nov) in myeloblastosis-associated virus type 1-induced nephroblastomas. Mol Cell Biol. 12:10-21,
- Keamey JF, Radbruch A, Liesegang B, Rajewsky K (1979) A new mouse myeloma cell line that has lost immunoglobulin Expression but permits the construction of antibody-secreting hybrid cell lines. J. Immunol. 123, 1548-50,
- Kocialkowski SY, H. Kingdom, J. Perbal, B. Schofield, PN (2001) Expression of the human NOV gene in first trimester fetal tissues. Anat Embryol, 203:417-427,
- Kumar S, Hand AT, Connor JR, et al. (1999) Identification and cloning of a Connective tissue growth factor-like cDNA from human osteoblasts encoding a novel regulator of osteoblast functions. J Biol Chem. 274:17123-17131,
- Lau L, Nathans D (1985) Expression of a set of growth-regulated immédiate early genes in BALB/c 3T3 cells: coordinate regulation with c-fos or c-myc. Proc Natl Acad Sci USA. 84: 1182-1186,
- Lin CJ, Leu S-J, Chen N, Tebeau CM, Lin S-X, Yeung C-H, Lau LJ, (2003) CCN3 (NOV) is a novel angiogenic regulator of the CCN protein family. J. Biol. Chem., 278, 24200-24208,
- Martinerie C, Gicquel C, Louvel A, Laurent M, Schofield P, LeBouc Y (2001) Altered expression of NovH is asociated with human adrenocortical tumorigenesis. JCEM. 86:3929-3940,
- Martinerie C, Huff V, Joubert I, et al. (1994) Structural analysis of the human nov proto-oncogene and expression in Wilms tumor. Oncogene 9: 2729-2732,
- Martinerie C, Perbal B (1991) Expression of a gene encoding a novel IGF binding protein in human tissues C R Acad Sci Paris. 313: 345-351,
- O'Reilly et al. (1997) Cell 88:277-285,
- Ortéga N, Hutchings H, Plouët J (1999) Signal relays in the VEGF system. Front. Biosc., 4, D141-D152,
- Pennica D, Swanson TA, Welsh JW, et al. (1998) WISP genes are members of the connective tissue growth factor family that are up-regulated in human colon tumors. Proc Natl Acad Sci. 95:14717-14722,
- Perbal B, Martinerie C, Sainson R, Werner M, He B, Roizman B (1999) The C-terminal domain of the regulatory protein NOVH is sufficient to promote interaction with fibulin 1C: a clue for a role of NOVH in cell- adhesion signaling. Proc Natl Acad Sci USA. 96: 869-874,
- Plouët J, Moro F, Coldeboeuf N, Bertagnolli S, Clamens S, Bayard F (1997) Extracellular cleavage of the vascular endothelial growth factor 189 aa form by urokinae is required for its mitogenic activity. J. Biol. Chem., 272, 13390-13396,
- Snaith M, Natarajan D, Taylor L, et al. (1996) Genomic structure and chromosomal mapping of the mouse nov gene. Genomics. 38: 425-428,
- Thibout H, Martinerie C, Creminon C, Godeau F, Boudou P, Le Bouc Y, Laurent M (2003) Characterization of NOVH in biological fluids: an enzyme immuno assay for the quantification of NOVH in sera from patients with diseases of the adrenal gland and of the nervous system. J Clin Endocrinol Metab. 88(1):327-336,
- Ying Z, Ling ML (1996) Isolation and characterization of xnov, a Xenopus laevis ortholog of the chicken nov gene. Gene. 17 1:243-248,

## Revendications

1. Utilisation :
- d'une protéine **caractérisée en ce qu'**elle est constituée par :
* la protéine NOV, représentée par la séquence SEQ ID NO : 2, ou
un fragment de cette protéine, représenté par la séquence SEQ ID NO : 12,
- d'une séquence nucléotidique **caractérisée en ce qu'**elle comprend ou est constituée par une séquence nucléotidique codant :
* soit pour la protéine NOV telle que définie ci-dessus,
* soit pour un fragment de la protéine NOV tel que défini ci-dessus,
ladite séquence nucléotidique correspondant à la séquence nucléotidique SEQ ID NO : 1 codant pour SEQ ID NO : 2, ou à la séquence SEQ ID NO : 11 codant pour SEQ ID NO : 12,
- d'un anticorps anti-idiotypique de la protéine NOV représentée par la séquence SEQ ID NO: 2, ledit anticorps mimant les fonctions de la protéine NOV en reconnaissant le VEGF,
pour la préparation d'un médicament destiné au traitement :
■ de pathologies nécessitant l'inhibition de la prolifération endothéliale, dans le cadre des pathologies suivantes : la dégénérescence maculaire liée à l'âge, les rétinopathies diabétiques, la polyarthrite rhumatoïde, les angiomes, les angiosarcomes, en particulier la maladie de Castelman et le sarcome de Kaposi, ou
■ de pathologies nécessitant l'inhibition de l'activation endothéliale, dans le cadre des pathologies suivantes le rejet d'allogreffe et de xénogreffe, les acrocyanoses, les sclérodermies, ou dans le cadre de la préparation de greffons entre le prélèvement et la transplantation.

2. Utilisation selon la revendication 1, d'une protéine **caractérisée en ce qu'**elle est constituée par :
* la protéine NOV, représentée par la séquence SEQ m NO : 2, ou
* un fragment de cette protéine, représenté par la séquence SEQ ID NO : 12,
pour la préparation d'un médicament destiné au traitement :
■ de pathologies nécessitant l'inhibition de la prolifération endothéliale, dans le cadre des pathologies suivantes : la dégénérescence maculaire liée à l'âge, les rétinopathies diabétiques, la polyarthrite rhumatoïde, les angiomes, les angiosarcomes, en particulier la maladie de Castelman et le sarcome de Kaposi, ou
■ de pathologies nécessitant l'inhibition de l'activation endothéliale, dans le cadre des pathologies suivantes : le rejet d'allogreffe et de xénogreffe, les acrocyanoses, les sclérodermies, ou dans le cadre de la préparation de greffons entre le prélèvement et la transplantation.

3. Utilisation selon la revendication 2, d'une protéine **caractérisée en ce qu'**elle est constituée par la protéine NOV, représentée par la séquence SEQ ID NO : 2.

4. Utilisation selon la revendication 2, d'une protéine **caractérisée en ce qu'**elle est constituée par un fragment de la protéine NOV, représentée par la séquence SEQ ID NO : 2, ledit fragment étant représenté par la séquence SEQ ID NO : 12

5. Utilisation d'une protéine **caractérisée en ce qu'**elle est constituée par un fragment de la protéine NOV, ledit fragment étant représenté par la séquence SEQ ID NO : 12, pour la préparation d'un médicament destiné au traitement des cancers.

6. Composition pharmaceutique **caractérisée en ce qu'**elle contient à titre de substance active :
- une protéine **caractérisée en ce qu'**elle est constituée par un fragment de la protéine NOV, représentée par la séquence SEQ ID NO : 2, ledit fragment étant représenté par la séquence SEQ ID NO : 12,
- une séquence nucléotidique **caractérisée en ce qu'**elle comprend ou est constituée par une séquence nucléotidique codant :
* soit pour la protéine NOV telle que définie ci-dessus,
* soit pour un fragment de la protéine NOV tel que défini ci-dessus,
ladite séquence nucléotidique correspondant à la séquence nucléotidique SEQ ID NO : 1 codant pour SEQ ID NO : 2, ou à la séquence SEQ ID NO : 1 codant pour SEQ ID NO : 12
- un anticorps anti-idiotypique de la protéine NOV représentée par la séquence SEQ ID NO: 2, ledit anticorps mimant les fonctions de la protéine NOV en reconnaissant le VEGF,
en association avec un vecteur pharmaceutiquement acceptable.

7. Composition pharmaceutique selon la revendication 6, **caractérisée en ce qu'**elle contient à titre de substance active une protéine **caractérisée en ce qu'**elle est constituée par un fragment de la protéine NOV, représentée par la séquence SEQ ID NO : 2, ledit fragment étant représenté par la séquence SEQ ID NO : 12, en association avec un vecteur pharmaceutiquement acceptable.

8. Utilisation selon la revendication 1 à 5, pour la préparation d'une composition selon l'une des revendications 6 à 7, destinée à être administrée à raison d'environ 0,1 à environ 20 mg/kg/jour.

## Claims

1. Use:
- of a protein **characterized in that** it is constituted by:
* the NOV protein, represented by the sequence SEQ ID NO: 2, or
* a fragment of this protein, represented by the sequence SEQ ID NO: 12,
- of a nucleotide sequence **characterized in that** it comprises or is constituted by a nucleotide sequence coding:
* either for the NOV protein as defined above,
* or for a fragment of the NOV protein as defined above,
said nucleotide sequence corresponding to the nucleotide sequence SEQ ID NO: 1 coding for SEQ ID NO: 2, or to the sequence SEQ ID NO: 11 coding for SEQ ID NO: 12,
- of an anti-idiotypic antibody of the NOV protein, represented by the sequence SEQ ID NO: 2, said antibody mimicking the functions of the NOV protein by recognizing VEGF,
for the preparation of a medicament intended for the treatment:
- of pathologies requiring the inhibition of endothelial proliferation, within the framework of the following pathologies: age-related macular degeneration, diabetic retinopathy, rheumatoid arthritis, angiomas, angiosarcomas, in particular Castelman's disease and Kaposi's sarcoma, or
- of pathologies requiring the inhibition of endothelial activation within the framework of the following pathologies: allograft and xenograft rejection, acrocyanosis, scleroderma, or within the framework of the preparation of grafts between collection and transplantation.

2. Use according to claim 1, of a protein **characterized in that** it is constituted by:
* the NOV protein, represented by the sequence SEQ ID NO: 2, or
* a fragment of this protein, represented by the sequence SEQ ID NO: 12,
for the preparation of a medicament intended for the treatment:
- of pathologies requiring the inhibition of endothelial proliferation, within the framework of the following pathologies: age-related macular degeneration, diabetic retinopathy, rheumatoid arthritis, angiomas, angiosarcomas, in particular Castelman's disease and Kaposi's sarcoma, or
- of pathologies requiring the inhibition of endothelial activation, within the framework of the following pathologies: allograft and xenograft rejection, acrocyanosis, scleroderma, or within the framework of the preparation of grafts between collection and transplantation.

3. Use according to claim 2, of a protein **characterized in that** it is constituted by the NOV protein, represented by the sequence SEQ ID NO: 2.

4. Use according to claim 2, of a protein **characterized in that** it is constituted by fragment of the NOV protein, represented by the sequence SEQ ID NO: 2, said fragment being represented by the sequence SEQ ID NO: 2.

5. Use of a protein **characterized in that** it is constituted by a fragment of the NOV protein, said fragment being represented by the sequence SEQ ID NO: 12, for the preparation of a medicament intended for the treatment of cancer.

6. Pharmaceutical composition **characterized in that** it contains as active ingredient:
- a protein **characterized in that** it is constituted by a fragment of the NOV protein, represented by the sequence SEQ ID NO: 2, said fragment being represented by the sequence SEQ ID NO: 12, or
- a nucleotide sequence **characterized in that** it comprises or is constituted by a nucleotide sequence coding:
* either for the NOV protein as defined above,
* or for a fragment of the NOV protein as defined above,
said nucleotide sequence corresponding to the nucleotide sequence SEQ ID NO: 1 coding for SEQ ID NO: 2, or to the sequence SEQ ID NO: 11 coding for SEQ ID NO: 12, or
- an anti-idiotypic antibody of the NOV protein, represented by the sequence SEQ ID NO: 2, said antibody mimicking the functions of the NOV protein by recognizing VEGF,
in combination with a pharmaceutically acceptable vector.

7. Pharmaceutical composition according to claim 6, **characterized in that** it contains as active ingredient a protein **characterized in that** it comprises or is constituted by a fragment of the NOV protein, represented by the sequence SEQ ID NO: 2, said fragment being represented by the sequence SEQ ID NO: 12, or in combination with a pharmaceutically acceptable vector.

8. Use according to claims 1 to 5, for the preparation of a composition according to one of claims 6 to 7, intended to be administered at a rate of approximately 0.1 to approximately 20 mg/kg/day.

## Patentansprüche

1. Verwendung:
- eines Proteins, **dadurch gekennzeichnet, dass** es aus Folgendem besteht:
* dem Protein NOV, das durch die Sequenz SEQ ID NO: 2 dargestellt wird, oder
* einem Fragment dieses Proteins, das durch die Sequenz SEQ ID NO: 12 dargestellt wird,
- einer Nukleotidsequenz, **dadurch gekennzeichnet, dass** sie eine Nukleotidsequenz umfasst oder aus ihr besteht, die:
* entweder für das Protein NOV, wie oben definiert,
* oder für ein Fragment des Proteins NOV, wie oben definiert,
kodiert,
wobei die Nukleotidsequenz der nukleotidsequenz SEQ ID NO: 1, die für SEQ ID NO: 2 kodiert, oder der Sequenz SEQ ID NO: 11 entspricht, die für die SEQ ID NO: 12 kodiert,
- einem anti-idiotypischen Antikörper des Proteins NOV, der durch die Sequenz SEQ ID NO: 2 dargestellt wird, wobei der Antikörper die Funktionen des Proteins NOV zur Erkennung des VEGF nachahmt,
zur Herstellung eines Medikaments zur Behandlung:
■ von Pathologien, bei denen die Hemmung der Endothelproliferation erforderlich ist, im Rahmen der folgenden Pathologien: alterbedingter Makuladegeneration, diabetischen Retinopathien, rheumatoider Polyarthritis, Angiomen, Angiosarkomen, insbesondere der Castelman-Krankheit und dem Kaposi-Sarkom, oder
■ von Pathologien, bei denen die Hemmung der Endothelaktivierung erforderlich ist, im Rahmen der folgenden Pathologien: Allotransplantat- und Xenotransplantatabstoßung, Akrozyanose, Sklerodermien oder im Rahmen der Herstellung von Transplantaten zwischen der Entnahme und der Transplantation.

2. Verwendung eines Proteins nach Anspruch 1, **dadurch gekennzeichnet, dass** es aus Folgenden besteht:
* dem Protein NOV, das durch die Sequenz SEQ ID NO: 2 dargestellt wird, oder
* einem Fragment dieses Proteins, das durch die Sequenz SEQ ID NO: 12 dargestellt wird,
zur Herstellung eines Medikaments zur Behandlung:
■ von Pathologien, bei denen die Hemmung der Endothelproliferation erforderlich ist, im Rahmen der folgenden Pathologien: alterbedingter Makuladegeneration, diabetischen Retinopathien, rheumatoider Polyarthritis, Angiomen, Angiosarkomen, insbesondere der Castelman-Krankheit und dem Kaposi-Sarkom, oder
■ von Pathologien, bei denen die Hemmung der Endothelaktivierung erforderlich ist, im Rahmen der folgenden Pathologien: Allotransplantat- und Xenotransplantatabstoßung, Akrozyanose, Sklerodermien oder im Rahmen der Herstellung von Transplantaten zwischen der Entnahme und der Transplantation.

3. Verwendung eines Proteins nach Anspruch 2, **dadurch gekennzeichnet, dass** aus dem Protein NOV besteht, das durch die Sequenz SEQ ID NO: 2 dargestellt wird.

4. Verwendung eines Proteins nach Anspruch 2, **dadurch gekennzeichnet, dass** es aus einem Fragment des Proteins NOV besteht, das durch die Sequenz SEQ ID NO: 2 dargestellt wird, wobei das Fragment durch die Sequenz SEQ ID NO: 12 dargestellt wird.

5. Verwendung eines Proteins, **dadurch gekennzeichnet, dass** es aus einem Fragment des Proteins NOV besteht, wobei das Fragment durch die Sequenz SEQ ID NO: 12 dargestellt wird, zur Herstellung eines Medikaments zur Behandlung von Krebs.

6. Pharmazeutische Zusammensetzung, **dadurch gekennzeichnet, dass** sie als Wirkstoff Folgendes enthält:
- ein Protein, **dadurch gekennzeichnet, dass** es aus einem Fragment des Proteins NOV besteht, das durch die Sequenz SEQ ID NO: 2 dargestellt wird, wobei das Fragment durch die Sequenz SEQ ID NO: 12 dargestellt wird, oder
- eine Nukleotidsequenz, **dadurch gekennzeichnet, dass** sie eine Nukleotidsequenz umfasst oder aus ihr besteht, die:
* entweder für das Protein NOV, wie oben definiert,
* oder für ein Fragment des Proteins NOV, wie oben definiert, kodiert,
wobei die Nukleotidsequenz der Nukleotidsequenz SEQ ID NO: 1, die für SEQ ID NO: 2 kodiert, oder der Sequenz SEQ ID NO: 11 entspricht, die für die SEQ ID NO: 12 kodiert, oder
- einen anti-idiotypischen Antikörper des Proteins NOV, der durch die Sequenz SEQ ID NO: 2 dargestellt wird, wobei der Antikörper die Funktionen des Proteins NOV zur Erkennung des VEGF nachahmt,
in Kombination mit einem pharmazeutisch verträglichen Vektor.

7. Pharmazeutische Zusammensetzung nach Anspruch 6, **dadurch gekennzeichnet, dass** sie als Wirkstoff ein Protein, das **dadurch gekennzeichnet ist, dass** es aus einem Fragment des Proteins NOV besteht, das durch die Sequenz SEQ ID NO: 2 dargestellt wird, wobei das Fragment durch die Sequenz SEQ ID NO: 12 dargestellt wird, in Kombination mit einem pharmazeutisch verträglichen Vektor enthält.

8. Verwendung nach Anspruch 1 bis 5 zur Herstellung einer Zusammensetzung nach einem der Ansprüche 6 bis 7 zur Verabreichung in einer Menge von etwa 0,1 bis etwa 20 mg/kg/Tag.
